# EUROPÄISCHE PATENTSCHRIFT

(11) **EP 3 220 721 B1**
(45) Veröffentlichungstag und Bekanntmachung des Hinweises auf die Patenterteilung: **29.11.2023**
(21) Anmeldenummer: 16202495.4
(22) Anmeldetag: 06.12.2016
(51) Int. Cl.: H05B 47/16, H05B 47/125, H05B 45/20, A61N 5/06

(54) **VERFAHREN ZUR EINSTELLUNG EINES LICHTPROFILS UND EIN BELEUCHTUNGSSYSTEM ZUR DURCHFÜHRUNG DES VERFAHRENS**
METHOD FOR ADJUSTING A LIGHT PROFILE AND LIGHTING SYSTEM FOR EXECUTING THE METHOD
PROCÉDÉ DE RÉGLAGE D'UN PROFILÉ LUMINEUX ET SYSTÈME D'ÉCLAIRAGE DESTINÉ À EXÉCUTER LEDIT PROCÉDÉ

(30) Priorität: 13.03.2016 DE 102016002962
(43) Veröffentlichungstag der Anmeldung: 20.09.2017
(73) Patentinhaber: Trilux GmbH & Co. KG, 59759 Arnsberg (DE)
(72) Erfinder: Dr. Knoche, Sebastian, 59757 Arnsberg (DE); Rudolph, Horst, 59505 Bad Sassendorf (DE); Appelhans, Silke, 58675 Hemer (DE); Köhler, Dennis, 44575 Castrop-Rauxel (DE)
(74) Vertreter: Lippert Stachow Patentanwälte Rechtsanwälte

(56) Entgegenhaltungen:
- EP-A1- 2 796 166
- DE-A1-102012 009 581
- JP-A- 2011 023 339
- US-A1- 2011 084 614

## Beschreibung

Die Erfindung betrifft ein Verfahren zur Einstellung eines Lichtprofils und ein Beleuchtungssystem zur Durchführung eines solchen Verfahrens.

Es ist seit langem bekannt, dass der Organismus von Lebewesen durch circadiane Rhythmen geprägt ist. Dies gilt beispielsweise für den Schlaf-Wach-Rhythmus, die Herzfrequenz, den Blutdruck sowie die Körpertemperatur. Derartige Abläufe können sich einem 24-Stundenzyklus anpassen, indem sie auf äußere Zeitgeber reagieren, d.h. von diesen synchronisiert werden. Als wichtigster Synchronisationsparameter für den Menschen in Bezug auf den Schlaf-Wach-Rhythmus gilt das Licht mit seinen im Laufe des Tag-Nacht-Rhythmus wechselnden Eigenschaften, insbesondere bezüglich der Helligkeit sowie der Lichtfarbe. Der Schlaf-Wach-Rhythmus des Menschen wird im Wesentlichen durch Ausschüttung/Nichtausschüttung des Hormons Melatonin gesteuert, was von der Tageszeit und der Helligkeit des vom Menschen über die Augen wahrgenommenen Lichtes abhängt. Weitere Untersuchungen haben ergeben, dass nicht nur die Helligkeit, sondern auch die sich im Tagesablauf ändernde Farbtemperatur des natürlichen Lichtes einen Einfluss auf den Melatoninspiegel im Blut aufweist. Es wurde insbesondere herausgefunden, dass Licht im Bereich von etwa 480 bis 490 nm eine Melatoninsuppression im Organismus hervorruft, sodass der Organismus auf "Aktivität" eingestellt wird. Demgegenüber wird dem Organismus durch einen erhöhten Melatoninspiegel am Abend oder bei Nacht das Einstellen eines verminderten Stoffwechsels signalisiert. Insofern wird dem menschlichen Organismus über die Ausschüttung von Melatonin auf hormonellem Wege der circadiane Schlaf-Wach-Rhythmus aufgeprägt.

Aufgrund des beschriebenen Sachverhaltes spielt es für das Wohlbefinden und die Leistungsfähigkeit des Menschen, der bei den meisten Tätigkeiten Kunstlicht ausgesetzt ist, eine wesentliche Rolle, die künstliche Beleuchtung mit Blick auf den circadianen Rhythmus, die Art der Tätigkeit und die aktuelle Tageszeit anzupassen. Dabei kann die Tätigkeit beruflicher oder privater Art sein kann und entsprechend in beruflicher oder privater Umgebung stattfinden.

Die Offenlegungsschrift EP 2796166 A1 betrifft ein Verfahren zur personalisierten Lichtsteuerung, bei dem ein personalisierter Ausgangssteuerparameter zum Steuern einer Lichtausgabe einer Lampe bereitgestellt wird. Der Steuerparameter umfasst dabei einen Farbtemperatur-Steuerparameter und wird auf der Grundlage eines, die Lokalzeit des Tages repräsentierenden Zeitparameters, eines Personentyp-Parameters und eines Personengeschlechtstyp-Parameters festgelegt. Dabei wird bei dem offenbarten Verfahren eine vorgegebene Zeitabhängigkeit der Farbtemperatur der Lichtausgabe der Lampe festgelegt, wobei die Tageslichtkurve der Farbtemperatur als Referenzlichtkurve für das beschriebene Beleuchtungssystem herangezogen werden kann. Die Offenlegungsschrift JP 2011023339 A betrifft ein Beleuchtungssystem mit einer Steuereinrichtung zur Steuerung der Beleuchtungsstärke und der Farbtemperatur. Das Beleuchtungssystem weist dabei eine Beleuchtungseinrichtung mit einer Lichtquelle und einer Steuereinrichtung zur Steuerung der Lichtquelle auf. Die Steuereinrichtung umfasst einen Taktgeber, eine Einrichtung zur Steuerung der Farbtemperatur in Abhängigkeit einer auf der Taktung des Taktgebers basierenden Zeiteinheit und eine Beleuchtungsstärke-Steuereinrichtung, welche die Steuerung der Veränderung der Beleuchtungsstärke der Lichtquelle in Abhängigkeit der vergangenen Zeit durchführt, die auf der Grundlage der Taktung ermittelt wird. Die Offenlegungsschrift DE 102012009581 A1 betrifft eine Vorrichtung sowie ein Verfahren zur Beleuchtung von Räumen, wobei mittels einer ersten Lichtquelle Licht mit einem ersten Lichtspektrum erzeugt und mittels einer zweiten Lichtquelle Licht mit einem zweiten Lichtspektrum erzeugt wird, und wobei durch Überlagerung des Lichtes der ersten Lichtquelle mit dem Licht der zweiten Lichtquelle ein Licht mit einem dritten Lichtspektrum nach einem beispielsweise durch natürliches Tageslicht vorgegebenen Muster erzeugbar ist. Die Offenlegungsschrift US 2011/0084611 A1 betrifft das Gebiet der Beleuchtungseinrichtungen, insbesondere ein System und ein Verfahren zum Steuern von Leuchten zum genauen Abstimmen von Helligkeit- und Farbvorgaben, angepasst an das Sonnenlicht. Dabei wird die Umsetzung einer vorgegebenen Steuerung des Blaulichtanteils für eine Weißlichtemission gelehrt. Zeitliche Verläufe der Helligkeit bzw. der Farbtemperatur des emittierten Lichtes, die vom System umgesetzt werden, sind insbesondere in zugeordneten Tagesplänen abgelegt.

Der Erfindung liegt die Aufgabe zugrunde, die von einem Beleuchtungssystem abgegebene Beleuchtungscharakteristik auf die menschlichen Bedürfnisse besser einzustellen, als es bei herkömmlichen Beleuchtungssystemen der Fall ist.

Diese Aufgabe löst die vorliegende Erfindung verfahrensseitig mit einem Verfahren gemäß dem unabhängigen Patentanspruch 1, und zwar einem Verfahren zur Einstellung eines Lichtprofils in einem Beleuchtungssystem mit den Schritten:
Bereitstellen einer mittels Stützstellen angegebener Referenzkurve eines Tagesverlaufs eines helligkeitsrelevanten Beleuchtungsparameters, wobei die Stützstellen eine Mehrzahl von Basiszeiten und eine Mehrzahl von den Basiszeiten zugeordneten Basiswerten des helligkeitsrelevanten Beleuchtungsparameters umfassen, und Bereitstellen einer mittels Stützstellen angegebener Referenzkurve eines Tagesverlaufs eines spektrumrelevanten Beleuchtungsparameters, wobei die Stützstellen die Mehrzahl von Basiszeiten und eine Mehrzahl von den Basiszeiten zugeordneten Basiswerten des einen spektrumrelevanten Beleuchtungsparameters umfassen, jeweils für einen vorbestimmten Tag;
Bereitstellen einer korrespondierenden Mehrzahl von Basiszeiten, einer Mehrzahl von den Basiszeiten zugeordneten Basiswerten des helligkeitsrelevanten Beleuchtungsparameters und einer Mehrzahl von den Basiszeiten zugeordneten Basiswerten des spektrumrelevanten Beleuchtungsparameters, jeweils für zumindest einen anderen Tag;
Ermitteln eines mittels Stützstellen angegebenen Tagesverlaufs des helligkeitsrelevanten Beleuchtungsparameters und eines mittels Stützstellen angegebenen Tagesverlaufs des spektrumrelevanten Beleuchtungsparameters für jeden zumindest einen anderen Tag, wobei die jeweiligen Basiszeiten des vorbestimmten Tages und jeweilige Basiszeiten des anderen Tages, die jeweiligen Basiswerte des helligkeitsrelevanten Beleuchtungsparameters des vorbestimmten Tages und die jeweiligen Basiswerte des helligkeitsrelevanten Beleuchtungsparameters des anderen Tages sowie die jeweiligen Basiswerte des spektrumrelevanten Beleuchtungsparameters des vorbestimmten Tages und jeweilige Basiswerte des einen spektrumrelevanten Beleuchtungsparameters des anderen Tages aneinander zugeordnet werden und zwischenliegende Zeiten und zwischenliegende Werte interpoliert, insbesondere linear interpoliert werden, sodass die jeweiligen Basiszeiten bzw. die jeweiligen Basiswerte des vorbestimmten Tages und des jeweils anderen Tages achsenweise abgebildet werden, und sich der jeweilige Tagesverlauf durch abschnittsweises Stauchen, Strecken oder Verschieben der entsprechenden Referenzkurve des Tagesverlaufs des vorbestimmten Tages ergibt, und Steuern bzw. Regeln von Betriebsparametern des Beleuchtungssystems zum Einstellen des ermittelten Tagesverlaufs des helligkeitsrelevanten Beleuchtungsparameters und des spektrumrelevanten Beleuchtungsparameters für jeden zumindest einen anderen Tag.

Das erfindungsgemäße Verfahren versetzt den Benutzer eines Beleuchtungssystems in die Lage, mit vergleichsweise geringem Aufwand ausgehend von einem einzelnen Tagesverlauf eines helligkeitsrelevanten Beleuchtungsparameters und eines spektrumrelevanten Beleuchtungsparameters an einem vorbestimmten Tag die an anderen Tagen des Jahresverlaufes optimalen Tagesverläufe dieser Beleuchtungsparameter unter Berücksichtigung der zeitlichen Änderungen des natürlichen Lichtes bzw. unter Berücksichtigung des Einflusses des Installationsortes des Beleuchtungssystems auf das natürliche Licht zum Einstellen einer optimalen Beleuchtungscharakteristik des Beleuchtungssystems bereitzustellen.

Erfindungsgemäß kann auf mit wenig Aufwand durchzuführende Art nicht nur die jahreszeitliche Änderung des natürlichen Lichtes in Bezug auf die zeitlichen Variationen von Sonnenauf- und -untergang, sondern stattdessen durch die Berücksichtigung von sich zeitlich ändernden Basiswerten bezüglich des helligkeitsrelevanten Beleuchtungsparameters und des spektrumrelevanten Beleuchtungsparameters eine besonders gute Synchronisation der Beleuchtungscharakteristik des Beleuchtungssystems an das Tageslichtprofil unter Berücksichtigung anwendungsspezifischer Überlegungen erreicht werden. Als helligkeitsrelevanter, d.h. die Helligkeit bestimmender bzw. mitbestimmender Beleuchtungsparameter kann beispielsweise der Lichtstrom, die Beleuchtungsstärke oder die Lichtintensität eingesetzt werden. Als spektrumrelevanter, z.B. die spektrale Strahlungsverteilung bestimmender bzw. mitbestimmender Beleuchtungsparameter kann beispielsweise die Farbtemperatur bzw. die sogenannte ähnlichste Farbtemperatur ("correlated colour temperature, CCT") oder melanopischer Wirkfaktor des Lichtes eingesetzt werden. Diese helligkeitsrelevanten bzw. spektrumrelevanten Beleuchtungsparameter geben somit das spezifische Emissionsverhalten der jeweiligen Lichtquelle(n) an.

Es sei bemerkt, dass die in der Anmeldung verwendete Angabe "Tag" breit verstanden werden kann und allgemein einen Zeitraum bezeichnen kann, der zumindest 6 Stunden oder zumindest 1 Stunde umfasst. Insofern kann die Angabe "Tag" jedoch auch genau 24 Stunden meinen. Der jeweilige "andere Tag" kann dabei die gleiche Zeitdauer wie der "vorbestimmte Tag" umfassen.

Weitere erfindungsgemäße Merkmale sind in den abhängigen Patentansprüchen definiert und werden von der allgemeinen Beschreibung, der Figurenbeschreibung sowie in den Zeichnungen gestützt.

Zweckmäßigerweise kann bei dem erfindungsgemäßen Verfahren vorgesehen sein, die Übertragung des vorgegebenen Tagesverlaufs des helligkeitsrelevanten Beleuchtungsparameters und des spektrumrelevanten Beleuchtungsparameters am vorbestimmten Tag dadurch auf den anderen Tag durchzuführen, dass nach der Zuordnung der Basiszeiten bzw. Basiswerten die Werteparameter für die Zeiten im Tagesverlauf achsenweise abgebildet werden, sodass sich die gesuchten Tagesverläufe letztlich durch abschnittsweises Stauchen bzw. Strecken der Zeitachse und der jeweiligen Werte- bzw. Parameterachse ergeben, wobei zwischen zwei Basiswerten liegenden Parameter interpoliert, insbesondere linear interpoliert werden können.

In einer besonderen Ausführungsform kann auch vorgesehen sein, zur Einstellung des Lichtprofils in einem Beleuchtungssystem neben dem zeitlichen Verlauf zumindest eines helligkeitsrelevanten Parameters auch die zeitlichen Verläufe mehrerer spektrumrelevanter Parameter wie beispielsweise die Farbtemperatur bzw. den Farbort, den melanopischen Wirkfaktor sowie unter Umständen weitere biologische Wirkfaktoren zu berücksichtigen, um eine noch bessere Anpassung des zeitlichen Verlaufs des Lichtes an die Bedürfnisse des jeweiligen Nutzers zu erreichen. Spektrumrelevante Parameter können dabei auch den Lichtfluss oder die Lichtintensitäten in gewissen spektralen Bändern angeben, z.B. innerhalb eines Bandbereichs von 380 nm und 385 nm oder 385 nm und 390 nm, etc. Das erfindungsgemäße Verfahren wird dann in entsprechender Weise durchgeführt, d.h. zu einen helligkeitsrelevanten Beleuchtungsparameter als auch zu den zumindest zwei spektrumrelevanten Parametern für einen vorbestimmten Tag werden die jeweiligen Tagesverläufe (Referenzkurven) bereitgestellt bzw. festgelegt. In diesen jeweiligen Referenzkurven werden Basiszeiten und Basisparameterwerte festgelegt. Gleichzeitig erfolgt eine Festlegung von Basiszeiten und Basisparameterwerten für zumindest einen, insbesondere mehrere über ein Jahr verteilte Tage, wobei die jeweiligen Basiszeiten und Basisparameterwerte sich von denen in der Referenzkurve im Allgemeinen zumindest teilweise unterscheiden. Basiszeiten können beispielsweise die Zeitpunkte für Sonnenaufgang, Mittagspause/Mittagsessen, Sonnenuntergang und Arbeitsende/Zubettgehen umfassen, während die Basiswerte für den helligkeitsrelevanten Parameter beispielsweise die Bedeutung maximal mögliche Helligkeit, Helligkeit zur Erfüllung einer Norm-Beleuchtungsstärke bei der Arbeit und nachtabgesenkte Helligkeit haben können. Auch bei Tätigkeit im Wohnbereich oder in der Freizeit kann die Festlegung vorgegebener Grenz- oder Schwellenwerte für helligkeits- und/oder spektrumrelevante Parameter wie z.B. Beleuchtungsstärken zweckmäßig sein, beispielsweise für das Kochen, Bügeln, Fernsehen etc. Je nach spezifischer Anwendung können auch noch weitere Basiszeiten und zugeordnete Basiswerte festgelegt werden, insbesondere um über den Tagesverlauf eine präzisere Einstellung eines gewünschten Lichtprofils zu erreichen.

Zur Ermittlung der jeweiligen Tagesverläufe des zumindest einen helligkeitsrelevanten Beleuchtungsparameters und der zumindest zwei spektrumrelevanten Beleuchtungsparameter werden die jeweiligen Referenzpunkte der Referenzkurven auf die "verzerrten" Kurvenpunkte der gesuchten Tagesverläufe abgebildet, wie obenstehend für den Fall, bei welchem ein einzelner spektrumrelevanter Parameter für das erfindungsgemäße Verfahren berücksichtigt wird, beschrieben wurde. Wie erläutert, werden zur Bestimmung der gesuchten Tagesverläufe die Referenzkurven abschnittsweise gestreckt, gestaucht oder verschoben. Zweckmäßigerweise werden dabei die jeweiligen Zeitachsen und die Parameterachsen für sich transformiert.

In einer Ausführungsform kann vorgesehen sein, die jeweiligen Tagesverläufe der beiden Beleuchtungsparameter funktional, insbesondere stückweise funktional anzugeben. In einer leicht umsetzbaren Ausführungsform kann jedoch vorgesehen sein, den Tagesverlauf des helligkeitsrelevanten Beleuchtungsparameters und des zumindest einen spektrumrelevanten Beleuchtungsparameters für den vorbestimmten Tag jeweils in Form einer Vielzahl von über den Zeitraum des Tages verteilten Stützstellen bereitzustellen, sodass sich durch die beschriebene Abbildung des Tagesverlaufs der beiden Beleuchtungsparameter für die gesuchten Tagesverläufe jeweils wiederum eine Vielzahl von über den Zeitraum des anderen Tages verteilte Stützstellen ergeben. Dabei kann die Anzahl der wie beschrieben ermittelten bzw. berechneten Stützstellen identisch sein mit der Anzahl der Stützstellen des vorbestimmten Tagesverlaufs.

Um eine möglichst durchgehende Ermittlung des Tagesverlaufs der Beleuchtungsparameter und damit eine kontinuierliche und nicht sprunghaft veränderliche Einstellung des Beleuchtungssystems bereitzustellen, kann zweckmäßigerweise vorgesehen sein, dass für Zeiten, die zwischen zwei Zeitenstützstellen liegen, eine Interpolation, insbesondere eine lineare Interpolation zur Ermittlung des jeweiligen Beleuchtungsparameters durchgeführt wird.

Zweckmäßigerweise kann vorgesehen sein, die Mehrzahl von Basiszeiten und/oder die Mehrzahl von Basiswerten des helligkeitsrelevanten Beleuchtungsparameters und/oder die Mehrzahl von Basiswerten des spektrumrelevanten Beleuchtungsparameters in Abhängigkeit vom Installationsort des Beleuchtungssystems festzulegen, sodass beispielsweise auch Änderungen in der Helligkeit bzw. der Farbtemperatur des natürlichen Lichtes im Jahreszyklus in einem Beleuchtungssystem zur Erzeugung einer vorgegebenen und an das natürliche Lichtprofil angepasste bzw. angelehnte Beleuchtungscharakteristik berücksichtigt werden können.

Die Anpassung der Beleuchtungscharakteristik des Beleuchtungssystems an die verschiedenen Gegebenheiten kann erfindungsgemäß auch auf Beleuchtungsrichtung und/oder Beleuchtungsfläche, bzw. Beleuchtungskegel erweitert werden, sodass auch weitere Beleuchtungsparameter, die für das Lichtempfinden eine Rolle spielen können, an den natürlichen Jahreszyklus angepasst werden können. Derartige zusätzliche Beleuchtungsparameter können wie vorstehend für den spektrumrelevanten Beleuchtungsparameter und den helligkeitsrelevanten Beleuchtungsparameter beschrieben verarbeitet werden, d.h. auch in diesen Fällen kann erfindungsgemäß für einen vorbestimmten Tag ein entsprechender Tagesverlauf bereitgestellt werden zusammen mit einer Mehrzahl von Basiszeiten bzw. Basiswerten für eine Mehrzahl von über das Jahr verteilten Tagen, sodass in gleicher Weise wie beschrieben zu jedem Tag auch diese Beleuchtungsparameter optimal angepasst werden können. Zu diesem Zweck kann das erfindungsgemäße Beleuchtungssystem weitere Einstelleinrichtungen aufweisen, wie beispielsweise verstellbare Umlenk- und/oder Fokussiereinrichtungen.

Es kann im Rahmen der Erfindung liegen, wenn jeweilige Basiswerte eines Beleuchtungsparameters an den über ein Jahr verteilten Tagen identisch sind, sodass in diesem Fall der Tagesverlauf des jeweiligen Beleuchtungsparameters allein in Bezug auf die Zeitachse, jedoch nicht in Bezug auf die Y-Achse gestaucht bzw. gestreckt werden kann.

Zweckmäßigerweise kann vorgesehen sein, dass in einem Beleuchtungssystem eine Mehrzahl von nutzungsspezifischen Tagesverläufen eines oder mehrerer Beleuchtungsparameter für einen vorbestimmten Tag zur Auswahl bereitgestellt werden, sodass ein Nutzer einen für die jeweilige Nutzung optimierten Verlauf bei der Initialisierung des Beleuchtungssystems auswählen kann. Beispielsweise können derartige Verläufe für Bürotätigkeiten, für Fabriktätigkeiten in einem Einschichtbetrieb, für einen Mehrschichtbetrieb, für einen Schulbetrieb unter Berücksichtigung der Mehrzahl von Pausen, für den Betrieb in einer medizinischen Einrichtung etc. bereitgestellt werden.

Um eine bestmögliche Steuerung der Betriebsparameter des Beleuchtungssystems auf die visuellen, biologischen und emotionalen Anforderungen des jeweiligen Benutzers zu erreichen, kann es zweckmäßigerweise sein, dass die Einstellung des Lichtprofils im Beleuchtungssystem durchgeführt wird auf der Grundlage vorgegebener Informationen bzw. Eingaben. Das erfindungsgemäße Verfahren kann somit im Unterschied zu herkömmlichen Verfahren zur Einstellung eines Lichtprofils in einem Beleuchtungssystem, welche das Durchlaufen fest vorgegebener Tagesverlaufskurven lehren, flexibel auf aktuelle Benutzungs- und Rahmenbedingungen des jeweiligen Beleuchtungssystems eingestellt werden. Insofern kann zweckmäßigerweise vorgesehen sein, dass die Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen in Abhängigkeit vom Installationsort des Beleuchtungssystems, in Abhängigkeit der Jahreszeit am Installationsort des Beleuchtungssystems und/oder in Abhängigkeit von meteorologischen bzw. klimatischen Daten, insbesondere Wetterdaten am Installationsort des Beleuchtungssystems für den jeweiligen Tag festgelegt werden. Beispielsweise können durch die Berücksichtigung von aktuellen Wetterdaten, bezogen aus dem Internet oder über Außensensoren, entsprechende Einflüsse auf die Lichtanforderungen berücksichtigt und zur Einstellung des erfindungsgemäßen Verfahrens herangezogen werden. Insbesondere können durch das Heranziehen dieser meteorologischen Daten Informationen über eine tagesaktuelle Dynamik des Tageslichtes, das beispielsweise von Wolkenmenge und Windstärke abhängt, gewonnen und zur Einstellung des erfindungsgemäßen Verfahrens herangezogen werden. Dabei kann davon ausgegangen werden, dass, sofern ein Raum Außenfenster aufweist, das Wetter Einfluss auf die Lichtbedürfnisse, beispielsweise in Bezug auf die präferierte Farbtemperatur sowie auf die melanopische Wirksamkeit, des Menschen besitzt, sodass dieser Einfluss beim erfindungsgemäßen Verfahren zur Einstellung des Lichtprofils in einem Beleuchtungssystem berücksichtigt werden kann. Beispielsweise können bei schönem Wetter in Anlehnung an einen blauen Himmel hohe Farbtemperaturen akzeptabel sein, während bei regnerischem und bewölktem Wetter niedrige Farbtemperaturen zum Abgleich an die jeweiligen Verhältnisse auch in Bezug auf das Lichtprofil des Beleuchtungssystems eingestellt werden können. Hierzu können beispielsweise die jeweiligen Basiswerte für die Farbtemperatur bei regnerischem und bewölktem Wetter zu niedrigen Werten verschoben werden. Je nach verwendeter Lichtquelle im Beleuchtungssystem sollte dann zweckmäßigerweise der melanopische Wirkfaktor nicht ebenso gesenkt werden, da das benötigte circadian wirksame Licht von der Lichtfarben-Präferenz entkoppelt ist.

Mit dem erfindungsgemäßen Verfahren kann das Lichtprofil eines Beleuchtungssystems auch an die Jahresdynamik des natürlichen Lichtes angepasst werden. Dieser Jahresverlauf, insbesondere die unterschiedliche Tageslänge, kann mit dem erfindungsgemäßen Verfahren in der künstlichen Beleuchtung nachgebildet werden. Beispielsweise können spezifische Basiszeiten an die Sonnenauf- und -untergangszeiten angepasst und das Lichtprofil so eingestellt werden, dass vor Sonnenauf- und nach Sonnenuntergang warme Lichtfarben im Beleuchtungssystem eingestellt werden. Auch in diesem Fall sollte der melanopische Wirkfaktor als spektrumrelevanter Beleuchtungsparameter nicht verändert werden, sondern auf der ihm zugedachten Tageskurve belassen werden. Neben der Berücksichtigung der Jahreszeit zur Festlegung der Basiszeiten bzw. Basiswerte der spektrumrelevanten Beleuchtungsparameter kann zweckmäßigerweise auch die geografische Lage des Beleuchtungssystems berücksichtigt werden, da auch diese einen Einfluss auf die Sonnenauf- und -untergangszeiten als auch auf die Tagesdynamik, z.B. das Mittags-Maximum, haben.

Zweckmäßigerweise kann auch die Umstellung zwischen Winter- und Sommerzeit bei der Festlegung der jeweiligen Basiszeiten bzw. spektrumrelevanten Beleuchtungsparameter berücksichtigt werden. Durch geeignete Anpassung der zeitlichen Verläufe des Lichtprofils in einem Zeitraum von einigen Tagen oder wenigen Wochen um die Zeitumstellung herum, kann den Menschen die Umstellung der inneren Uhr erleichtert werden, beispielsweise indem die bei der Zeitumstellung auftretenden Sprünge geglättet werden, insbesondere, dass jeden Tag um die Zeitumstellung herum eine leicht geänderte Tagesdynamik durchlaufen wird.

Zweckmäßigerweise kann das erfindungsgemäße Verfahren zur Einstellung eines Lichtprofils in einem Beleuchtungssystem auch in Abhängigkeit der sich im Wirkbereich des Beleuchtungssystems befindlichen Person bzw. Personen durchgeführt werden. Insofern kann vorgesehen sein, dass die Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen in Abhängigkeit von zumindest einer, sich im Wirkbereich des Beleuchtungssystems befindlichen Personen festgelegt wird. Diese Festlegung kann beispielsweise auf der Grundlage von objektiven Eigenschaften der zumindest einen Person wie Alter, Geschlecht, Bewegung oder subjektiven Eigenschaften bzw. Empfinden der zumindest einen Person, wie Lichtpräferenz, Stimmung oder Chronotyp durchgeführt werden, da derartige Parameter den jeweiligen Bedarf der jeweiligen Person in Bezug auf die Beleuchtung festlegen können. Dabei kann auch vorgesehen sein, dass bei Anwesenheit von mehr als einer Person eine Mittelung der anfallenden Werte, die Wahl einer dominierenden Eigenschaft, die auf die Mehrheit der anwesenden Personen zutrifft, oder auch die Berücksichtigung der Werte zur Ermittlung eines Extremwerts als maßgebend für die beschriebene Festlegung der Mehrzahl der Basiszeiten und/oder der Mehrzahl von Basiswerten des helligkeitsrelevanten Beleuchtungsparameters und/oder die Mehrzahl von Basiswerten zumindest eines spektrumrelevanten Beleuchtungsparameters durchgeführt wird. Beispielsweise kann das erfindungsgemäße Verfahren auf das Alter der im Raum befindlichen Personen abgestellt werden, insbesondere dadurch, dass die Mehrzahl von Basiswerten des helligkeitsrelevanten Beleuchtungsparameters grundsätzlich erhöht werden, beispielsweise über einen vorgegebenen Faktor, welcher über alle Basiszeiten konstant sein kann. Dabei kann z.B. berücksichtigt werden, dass mit zunehmendem Alter die Trübung der Linse insbesondere im höherfrequenten Bereich des visuellen Spektrums zunimmt und es zu einer Gelbverschiebung des subjektiv erfassten Spektrums kommt. Im blauen Bereich sind jedoch die melanopischen Lichtwirkungen am größten. Um dieser altersabhängigen Reduzierung des Transmissionsgrads entgegenzuwirken, kann die Intensität im blauen Bereich des Spektrums erhöht werden. Zudem ist eine altersabhängige Pupillenverkleinerung bekannt, die im Rahmen der Erfindung auch dadurch berücksichtigt werden kann, dass Basiswerte des helligkeitsrelevanten Beleuchtungsparameters erhöht werden.

Das erfindungsgemäße Verfahren kann insbesondere auch über die Steuerung des Lichtprofils genutzt werden, um persönliche Stimmungen zu bedienen oder eher entgegenzuwirken. In ähnlicher Weise kann es zweckmäßig sein, den Chronotyp der jeweiligen Person bzw. Personen für die beschriebene Festlegung heranzuziehen, da der Chronotyp einen direkten Einfluss auf den individuellen Tagesverlauf, insbesondere in Bezug auf einen Offset bezüglich des allgemeinen lokalen Tagesverlaufs, der sich nach Sonnenauf- und -untergang richten kann, darstellt. Im einfachsten Fall kann das erfindungsgemäße Verfahren so angepasst werden, dass bestimmte Basiszeiten je nach Chronotyp mit einem positiven oder negativen Offset versehen werden.

Auch die Berücksichtigung von kulturellen Parametern zur Festlegung von Basiszeiten bzw. Basiswerten kann zweckmäßig sein. Beispielsweise werden in den nördlichen Gebieten warme Lichtfarben bevorzugt, in südlichen Gebieten kältere, sodass zweckmäßigerweise die Tagesverlaufs-Kurve für die Lichtfarbe mit einem positiven oder negativen Offset beaufschlagt werden kann. Zweckmäßigerweise kann auch vorgesehen sein, dass zumindest eine objektive oder subjektive Eigenschaft bzw. Empfinden der zumindest einen Person mittels einer Datenverarbeitung von über eine Sensoreinrichtung erfassten Daten, insbesondere Bilddaten ermittelt wird.

Ganz allgemein können personenbezogene Daten von im Wirkbereich des Beleuchtungssystems befindlichen Personen durch einen oder mehrere Sensoren, beispielsweise einem Personen-ID-Sensor oder einer Kamera mit nachfolgender Bildverarbeitung erfasst bzw. ermittelt werden.

Ferner kann die Erfassung und Berücksichtigung von kontextbezogenen Parametern zur Festlegung von Basiszeiten bzw. Basiswerten zweckmäßig sein. Hierzu können verwendete Sensoren beispielsweise auch zum Erkennen bestimmter Situationen ausgebildet sein, beispielsweise wie und wo sich eine Person im Raum bzw. Sensorbereich bewegt oder befindet. Beispielsweise können auf dieser Art unterschiedlichste Situationen wie Präsentation, Meeting, Pause, Bildschirmarbeit, Schreibtischarbeit, Ausführung bestimmter Tätigkeiten im Wohnbereich wie Kochen oder Bügeln, oder im Freizeitbereich etc. erfasst werden und vordefinierte Lichteinstellungen, respektive Basiswerte der helligkeitsrelevanten Beleuchtungsparameters und/oder Basiswerte des zumindest einen spektrumrelevanten Beleuchtungsparameters, die vorab in Bezug auf die genannten Tätigkeiten abgelegt wurden, entsprechend aufgerufen und automatisch zur Steuerung des Beleuchtungssystems im Ansprechen auf die mittels Sensorsignalen erkannten Situationen eingestellt werden.

Es versteht sich, dass eine solche Auswahl von, in Bezug auf die genannten Tätigkeiten abgelegte und vordefinierte Lichteinstellungen, respektive Basiswerte der helligkeitsrelevanten Beleuchtungsparameters und/oder Basiswerte des zumindest einen spektrumrelevanten Beleuchtungsparameters auch durch eine benutzerspezifische Eingabe zur Steuerung des Verfahrens vorgesehen sein kann. Hierzu können Taster und Steuersysteme, insbesondere umfassend Touchscreens, Gestensensoren und/oder auch eine Spracherkennung vorgesehen sein, um eine manuelle Wahl von Szenen und damit eine Einstellung von Helligkeit, Farbtemperatur und Spektrum auszuwählen.

Darüber hinaus können über eine Datenverbindung zu einem internen Netzwerk, zu mobilen Geräten oder zum Internet Informationen über aktuelle Nutzungsbedingungen aus Terminkalendern und Raumplänen abgerufen oder auf der Basis der in Reichweite befindlichen mobilen Geräten erkannt werden, insbesondere auch Informationen über die Anzahl und Identität der Teilnehmer, Thema des Termins etc. und zur Einstellung bzw. zur Auswahl geeigneter Basiszeiten, Beleuchtungsparameter bzw. Basiswerte herangezogen werden.

Auch können erfindungsgemäß Gegenstände im Wirkungsbereich des Beleuchtungssystems über Sensorik in ein IoT-(Internet der Dinge)-Netzwerk eingebunden werden und für die Informationsgewinnung genutzt werden. Beispielhaft sei hier der Einsatz von Drucksensoren in Sitzkissen genannt, um die Anzahl von Personen zu bestimmen, die um einen Tisch herum sitzen. Ein weiteres Beispiel ist die Informationsgewinnung bei Handhabung von Gegenständen (z. B. Nutzung eines Stiftes) .

Dabei ist es auch möglich, mit Methoden des Data-Minings, z.B. über Klassifikationsalgorithmen, zu ermitteln, welche Eigenschaften eines Datensatzes, wie ein Terminplan, auf welche einzustellenden Lichtprofile schließen lassen, wenn vorab Trainingsdaten aufgenommen wurden. Diese Trainingsdaten können Eigenschaften und zugehörige, manuell getroffene Lichteinstellungen enthalten, sodass aus diesen Rückschlüsse auf die Lichteinstellung bezüglich einer Szene gewonnen werden können. Eine implementierte künstliche Intelligenz kann erfindungsgemäß über Trainingsdaten hinaus aus späteren, im laufenden Betrieb getroffenen, manuellen Eingriffen in das System lernen und diese in die Generierung der Szenen mit einbinden.

Die jeweilige Nutzungssituation eines Raums kann dabei mit Hilfe der obenstehend schon angegebenen Sensoren und weiteren Sensoren erfasst werden. Neben Mikrofonen, die mit einer Spracherkennung verbunden sind, und Anwesenheits-/Präsenzdetektoren, können kamerabasierte Systeme für eine Bilderfassung genutzt werden. Mit Hilfe eines kamera-basierten Systems, das auf eine Bibliothek zur Gesichtserkennung zurückgreift, können Daten wie Alter, Geschlecht und Stimmung von im Raum befindlichen Personen aus der Situation erkannt und für die Festlegung der beschriebenen Basiszeiten, Beleuchtungsparameter bzw. Basiswerte der spektrumrelevanten Beleuchtungsparameter verwendet werden. Ferner kann über die Sensoren und eine optionale Datenverarbeitung der Kontext einer Situation bzw. eine Tätigkeit aus der Interaktion von Menschen untereinander geschlossen werden. Sind beispielsweise mehrere Personen anwesend und es ist leise, werden die Personen mit großer Wahrscheinlichkeit selbstständige, konzentrierte Arbeiten durchführen. Redet eine einzelne Person in Anwesenheit von vielen, findet ein Vortrag statt oder sie telefoniert. Für eine Differenzierung beider Situationen sind insofern weitere Daten erforderlich, zum Beispiel Information aus einem Terminkalender. Bei mehreren sprechenden Personen ist eine Diskussion oder Unterhaltung sehr wahrscheinlich.

Ferner können Geräte, die in das IoT eingebunden sind, untereinander kommunizieren und Daten austauschen, um Information zur Festlegung zu erhalten. Dadurch wird das Data Mining unterstützt und es ergeben sich Rückschlüsse auf den Kontext. So kann beispielsweise ein Beamer das Leuchtensystem darüber informieren, dass er eingeschaltet wurde und genutzt wird und sich nicht im Ruhemodus befindet. Es ist somit sehr wahrscheinlich, dass eine Präsentation stattfindet. Herd und Dunstabzugshaube können hingegen durch ihren Betrieb auf eine Kochsituation hinweisen.

In einer besonders einfachen Ausführungsform kann die Festlegung der beschriebenen Basiszeiten, Beleuchtungsparameter bzw. Basiswerte der spektrumrelevanten Beleuchtungsparameter auch statisch durchgeführt werden, beispielsweise dadurch, dass Werte vorgegebener Referenzkurven mit einem festen bzw. wählbaren Prozentwert multipliziert werden. So kann beispielsweise eine Szene zur Beleuchtung während einer Beamer-Präsentation eingestellt werden, bei der die Helligkeit im Raum zu Gunsten der Erkennbarkeit des projizierten Beamer-Bildes reduziert wird. Fallweise kann es sinnvoll sein, nur einen oder einen Teil der Parameter, beispielsweise nur den helligkeitsrelevanten Parameter statisch zu belassen und die anderen Parameter in ihrer Dynamik beizubehalten oder eine alternative Dynamik vorzusehen. Beispielsweise könnte im Falle einer Beamer-Präsentation die Helligkeit abgesenkt und statisch eingestellt sein, während die Farbtemperatur ihre Tagesdynamik beibehalten könnte, wobei der melanopische Wirkfaktor morgens, mittags und am frühen Nachmittag deutlich erhöht werden kann, um den biologischen Lichtbedarf zu dieser Zeit trotz geringerer Helligkeit zu decken.

In einer besonderen Ausführungsform kann es zweckmäßig sein, dass die Festlegung der Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen in Abhängigkeit zumindest einiger der oben stehend angegebenen Einflussgrößen durchgeführt wird. Beispielsweise kann es zweckmäßig sein, das erfindungsgemäße Einstellen des Lichtprofils eines Beleuchtungssystems vom Installationsort des Beleuchtungssystems, in Abhängigkeit der erfassten aktuellen Wetterdaten am Installationsort, in Abhängigkeit der mittels Sensoren erfassten Nutzungssituation in einem Raum und in Abhängigkeit der Altersstruktur der sich im Raum befindlichen Personen einzustellen. Dabei kann, wie oben stehend erläutert, dieser eine "Tag" grundsätzlich auch einen kürzeren Zeitraum umfassen, insbesondere einen Zeitraum, beispielsweise von wenigen Stunden.

Die Berücksichtigung einer Mehrzahl von Einflussgrößen zur Festlegung der Mehrzahl von Basiszeiten und/oder Basiswerten kann vorzugsweise mittels einer Datenverarbeitung so durchgeführt werden, dass die Berechnung kommutativ erfolgt, d.h. die Reihenfolge bei der Berücksichtigung der jeweiligen Einflussgröße ist nicht relevant. Eine solche Annahme kann die Festlegung der Mehrzahl von Basiszeiten und/oder Basiswerten wesentlich erleichtern, ohne zumindest in den meisten Fällen zu einer unzulässigen Verfälschung der tatsächlich richtigen Werte zu führen.

Die obige Aufgabe wird darüber hinaus gelöst durch ein Beleuchtungssytem gemäß dem unabhängigen Patentanspruch 11, J umfassend zumindest ein Betriebsgerät zum Speisen zumindest eines Leuchtmittels, wobei das Beleuchtungssystem eingerichtet ist, ein obenstehend beschriebenes Verfahren zur Einstellung eines Lichtprofils auszuführen. Dabei kann das erfindungsgemäße Beleuchtungssystem eine Betriebsgeräteeinrichtung, insbesondere mehrere Betriebsgeräte umfassen, die zum Betrieb von mehreren Leuchtmitteln zur Abgabe von Licht mit zueinander unterschiedlicher Farbtemperatur ausgebildet sein können, sodass die Farbtemperatur des Lichtgemischs des Beleuchtungssystem bzw. einer Leuchte des Beleuchtungssystem durch Einstellen der relativen Helligkeiten der Leuchtmittel einstellbar bzw. regelbar ist.

Die Erfindung wird im Folgenden durch das Beschreiben einer Ausführungsform sowie Abwandlungen hiervon unter Bezugnahme auf die beiliegenden Zeichnungen erläutert, wobei
- Figur 1: ein erfindungsgemäßes Beleuchtungssystem zur Durchführung des erfindungsgemäßen Verfahrens zur Einstellung eines Lichtprofils,
- Figur 2a-2c: einen beispielhaften Tagesverlauf für die Helligkeit sowie die Farbtemperatur eines Beleuchtungssystems für Büroräumlichkeiten (Fig. 2a)), eine Tabelle der Werte (Fig. 2c))sowie Maße für die biologische Wirkung (Fig. 2b)),
- Figur 3: einen anderen Tagesverlauf der Helligkeit an einem bestimmten Tage unter Angabe vorgegebener Basiszeiten und Basishelligkeitswerten,
- Figur 4a: eine tabellarische Ansicht der in Figur 3 angegebenen Basiszeiten und Basishelligkeiten für zwei unterschiedliche, zeitlich voneinander beabstandete Tage im Jahresverlauf,
- Figur 4b: den Verlauf zweier Basiszeiten t_set, t_rise über ein Jahr,
- Figur 5a: die Abbildung der Basiszeiten eines vorbestimmten Tages auf die Basiszeiten eines weiteren Tages,
- Figur 5b: die Abbildung der Basiswerte für die Helligkeit eines vorbestimmten Tages auf die Basiswerte der Helligkeit eines anderen Tages,
- Figur 6: den auf der Grundlage des erfindungsgemäßen Verfahrens ermittelten Tagesverlauf der Helligkeit für den anderen Tag,
- Figuren 7a, b, c bis 12a, b, c: weitere vorgegebene Tagesverläufe für die Helligkeit sowie die Farbtemperatur für unterschiedliche Anwendungen (a), eine jeweilige Tabelle der Werte (b) sowie Maße für die jeweilige biologische Wirkung (c),
- Figur 13: ein Ablaufdiagramm zur erfindungsgemäßen Einstellung eines Lichtprofils in einem Beleuchtungssystem,
- Figur 14: ein Detail zum Ablaufdiagramm gemäß Figur 13 bezüglich der Basiszeiten,
- Figur 15: ein Detail zum Ablaufdiagramm gemäß Figur 13 bezüglich eines helligkeitsrelevanten Beleuchtungsparameters,
- Figur 16: den Tagesverlauf des helligkeitsrelevanten Beleuchtungsparameters unter Berücksichtigung der Eigenschaft Alter, und
- Figur 17: den Tagesverlauf des helligkeitsrelevanten Beleuchtungsparameters unter Berücksichtigung der Anpassungen der Basiszeiten gemäß Figur 14 zeigt.

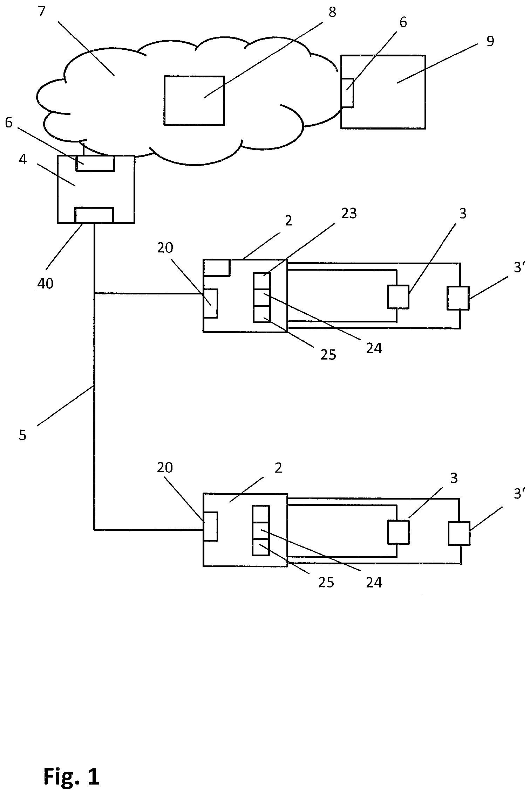

Ein erfindungsgemäßes Beleuchtungssystem zur Umsetzung des erfindungsgemäßen Verfahrens zur Einstellung eines Lichtprofils ist in einer Prinzipdarstellung in Figur 1 angegeben. Das Beleuchtungssystem umfasst hier eine Anzahl von Betriebsgeräten 2, die zum Betrieb von Leuchtmitteln ausgebildet sind, vorliegend zum Betreiben von LED-Einrichtungen 3, 3'. Diese LED-Einrichtungen 3, 3' sind zur Emission von Licht unterschiedlicher Wellenlänge ausgebildet, sodass die Farbtemperatur des Lichtgemischs einer Leuchte, bestehend im Wesentlichen aus dem Betriebsgerät 2 und den beiden von diesem gespeisten LED-Einrichtungen, durch Einstellen der relativen Helligkeiten, respektive Dimmpegeln zur Einstellung des jeweiligen LED-Stroms festlegbar ist.

Darüber hinaus ist die Gesamthelligkeit, respektive der Gesamtlichtstrom des von einer Leuchte, umfassend die beiden LED-Einrichtungen 3, 3', abgegebenen Lichtes durch synchrones Verändern des Dimmpegels beider LED-Einrichtungen 3, 3' einstellbar. Figur 1 zeigt insofern ein Beleuchtungssystem, umfassend zwei Leuchten, welche jeweils getrennt voneinander in Bezug auf die Farbtemperatur des abgegebenen Lichtes und in Bezug auf die Gesamthelligkeit einstellbar sind. Neben einer lokalen Steuereinrichtung 24 sind beide Leuchten über Steuerschnittstellen 20 an einen digitalen Bus 5 angeschlossen und werden so über eine zentrale Steuereinrichtung 4 mit Steuerinformation zum tages- und jahreszeitabhängigen Betrieb der Leuchten versorgt. In einer nicht dargestellten Ausführungsform kann die Datenverbindung zwischen den Leuchten und der zentralen Steuereinrichtung durch Vorsehen entsprechender Luftschnittstellen auch drahtlos realisiert sind.

In der beschriebenen Ausführungsform ist die zentrale Steuereinrichtung 4 über eine Netzwerkschnittstelle 6 an ein Computernetzwerk 7 angeschlossen und kann so auf einen zentralen Speicher 8 zugreifen, auf dem nutzungsspezifische Parameter zur Abrufung abgelegt werden können, beispielsweise über einen Server 9, der über eine Netzwerkschnittstelle 6 mit dem Computernetzwerk verbunden ist.

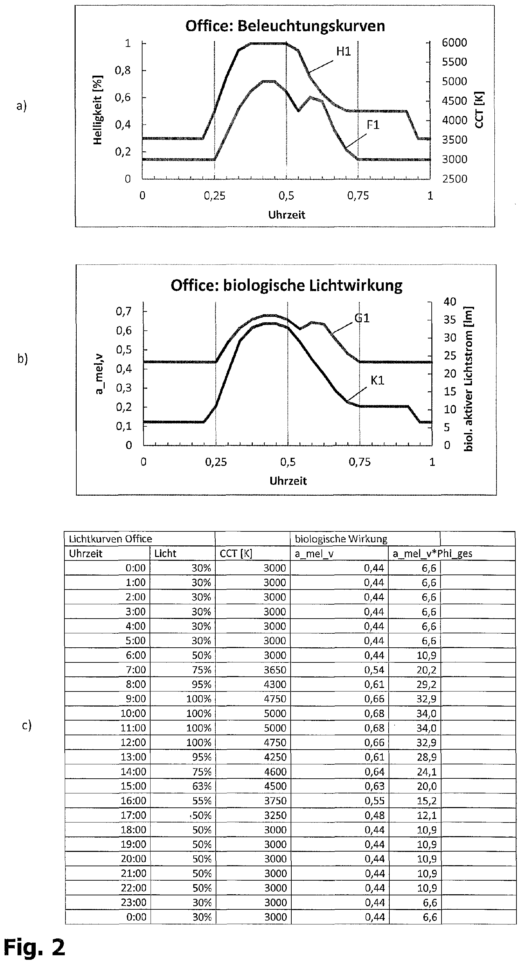

Mit Bezug auf die Figuren 2a, b wird im Folgenden ein erfindungsgemäßer Tagesverlauf der Helligkeit und der Farbtemperatur beschrieben, wie er für ein Büro zur Unterstützung des circadianen Rhythmus optimiert wurde. Während Figur 2a die Verläufe der beiden Beleuchtungsparameter zeigt, sind in Figur 2c Stützstellen der Verläufe angegeben. Die Kurven der Fig. 2b geben die Verläufe K1, G1 zweier Maße von biologischen Lichtwirkungen wieder. Erkennbar weist die Helligkeitskurve H1 drei Referenzwerte auf. Zwischen den Zeiten 0 Uhr bis 5 Uhr beträgt die Helligkeit 30% vom Maximalwert, beispielsweise für eine Reinigungstätigkeit, wobei nach den technischen Regeln der Arbeitsstätten (ASR) eine Beleuchtungsstärke von 100 lx vorliegen muss. Die Beleuchtungskurven gemäß Figur 2a sind so eingerichtet, dass gegen 6 Uhr der tageslichtsynchrone Teil der aktivierenden Beleuchtungskurve beginnt mit 50% der Helligkeit, die das Minimum der zu erfüllenden Beleuchtungsstärke darstellen, wobei die 50% eine normgerechte Beleuchtungsstärke für Bildschirmarbeit bereitstellen. Gegen 9 Uhr erreicht die Helligkeit ihr Maximum von 100%, gleichzeitig ist der maximale melanopische Wirkfaktor durch Einstellen einer Farbtemperatur von 4750 K erreicht. Gegen 12 Uhr ist durch entsprechende Wahl der Helligkeit und der Farbtemperatur der Beleuchtung bzw. des Lichtprofils ein mittlerer melanopischer Wirkfaktor zur leichten Entspannung zur Mittagspause und Erhöhung des Komforts eingestellt. Danach fällt die Helligkeitskurve H1, gleichzeitig steigt wieder die Farbtemperatur F1, um einem Mittagstief entgegenzuwirken. Gegen 16 Uhr wird durch das angegebene Einstellen der Helligkeit und der Farbtemperatur das Arbeitsende vorbereitet, wobei gegen 18 Uhr eine normgerechte Beleuchtung von 50% Helligkeit und geringer melanopischer Wirkung eingestellt ist. Zwischen 22 und 23 Uhr erfolgt dann wiederum die Einstellung auf ein Reinigungslicht mit einer Helligkeit von 30% und einer Farbtemperatur von 3.000 K.

Die mit Bezug auf die Figuren 2a - c beschriebenen Beleuchtungskurven H1, F1 können in dem in Figur 1 angegebenen Beleuchtungssystem beispielsweise von der zentralen Steuerung 4 durch Übermittlung entsprechender Steuersignale an die Betriebsgeräte 2 eingestellt werden, wobei hierzu die LED-Einrichtungen 3, 3' mit den jeweiligen entsprechenden Dimmpegeln zur Realisierung der Beleuchtungskurven H1, F1 gespeist werden. Derartige Beleuchtungskurven können der zentralen Steuereinrichtung in dem beschriebenen Beleuchtungssystem durch einen beispielsweise in einem Netzwerk 7 angeordneten Speicher zentral zur Verfügung gestellt werden, auf dem die zentrale Steuereinrichtung 4 über ihre Netzwerkschnittstelle 6 zugreifen kann, wobei aktualisierte Beleuchtungskurven für unterschiedliche Gegebenheiten über einen auch an das Netz angeschlossenen Server 9 im Speicher 8 abgelegt werden können.

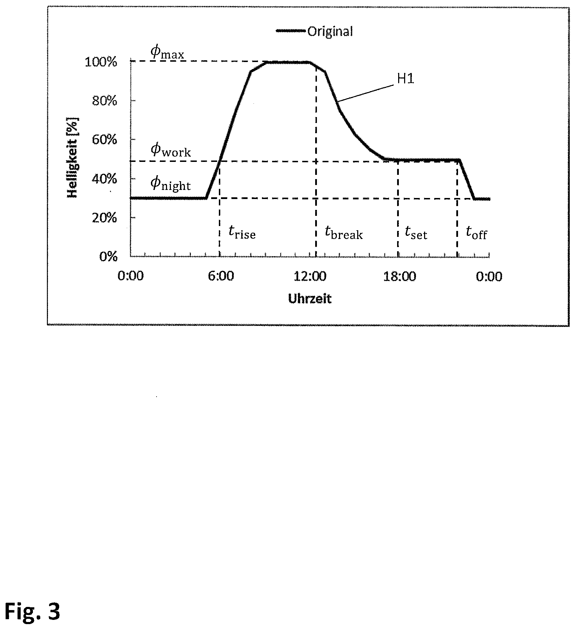

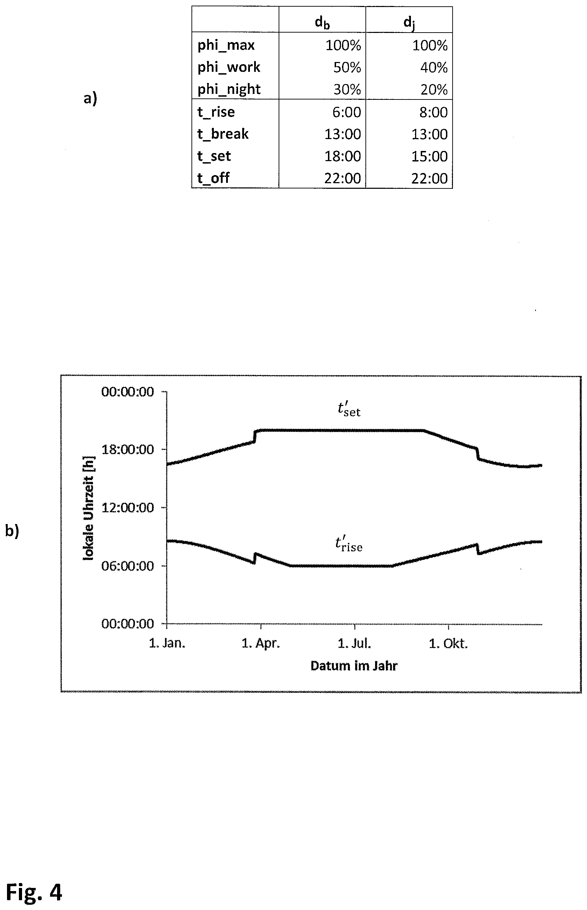

Erfindungsgemäß reicht es für den Betrieb des Beleuchtungssystems aus, für einen Tagesverlauf für einen vorgegebenen Tag die beschriebenen Beleuchtungskurven bereitzustellen. Das System ermittelt dann selbständig die angepassten Beleuchtungskurven für alle Tage des Jahres, sodass über den gesamten Jahreszyklus optimierte Beleuchtungskurven, hier in Bezug die Helligkeit und die Farbtemperatur eingestellt werden. Dieser Vorgang soll im Folgenden mit Bezug auf die Figuren 3 - 6 und in Bezug auf die beispielhafte Helligkeitskurve H1 der Figur 2a erläutert werden, die in Figur 3 nochmals wiedergegeben ist. Dabei soll die in Figur 3 angegebene Grundform der Tagesdynamik der Helligkeitskurve H1, die für einen bestimmten Tag des Jahres angegeben ist, über das gesamte Jahr beibehalten werden, jedoch unter Berücksichtigung gewisser jahresdynamischer Variationen zur Optimierung des Einflusses des Lichtes auf den menschlichen Organismus. Die in Figur 3 angegebene Helligkeitskurve H1 weist mehrere Basis- oder Schlüsselzeiten t_rise, t_break, t_set und t_off auf, darüber hinaus Basiswerte für die Helligkeit φ_night, φ_work und φ_max. Die Helligkeitsparameter φ_max, φ_work, φ_night sind an die gegebenen Umstände anzupassen, beispielsweise um sicherzustellen, dass bei der Helligkeit φ_work eine Normbeleuchtungsstärke von 500 lx für Bildschirmarbeit erreicht wird. Die angegebenen Basiszeiten weisen die typische Jahreszeitendynamik auf und können darüber hinaus auch vom geografischen Installationsort des Beleuchtungssystems abhängen. Zur Durchführung der Transformation des in Figur 3 angegebenen Tagesverlaufs ist in der beschriebenen Ausführungsform nicht nur dieser Tagesverlauf mittels einer Vielzahl von Stützstellen {φ,t} gespeichert, sondern auch für jeden Tag des Jahres die angegebenen Basiszeiten t_rise, t_break, t_set, t_off sowie die Basiswerte φ_night, φ_work und φ_max. Figur 4a zeigt beispielhaft die angegebenen Basiszeiten und Basisparameter für die Helligkeit für zwei unterschiedliche Tage d_b, d_j, wobei d_b der Tag sein soll, welcher der in Figur 3 angegebenen Helligkeitskurvenverlauf H1 entspricht. Erkennbar sind nicht nur Basiszeiten der beiden Datensätze unterschiedlich, sondern auch in Bezug die Basiswerte φ_work und φ_night.

Wie schon erläutert, ist das erfindungsgemäße Verfahren zur Einstellung eines Lichtprofils prinzipiell auf alle Beleuchtungssysteme und damit alle Tätigkeiten im Wirkungsbereich des jeweiligen Beleuchtungssystems anwendbar. In diesem Sinne können für derartige Tätigkeiten Schwellen wie φ_work, φ_bügeln, oder allgemein φ_activity, für jeweilige helligkeits- und/oder spektrumrelevante Parameter festgelegt werden.

Figur 4b zeigt einen beispielhaften jahreszeitlichen Verlauf für die beiden Basiszeiten t_set und t_rise. Die aus den Figuren ersichtlichen Sprünge resultieren durch die jeweilige Umstellung zwischen Sommer- und Winterzeit.

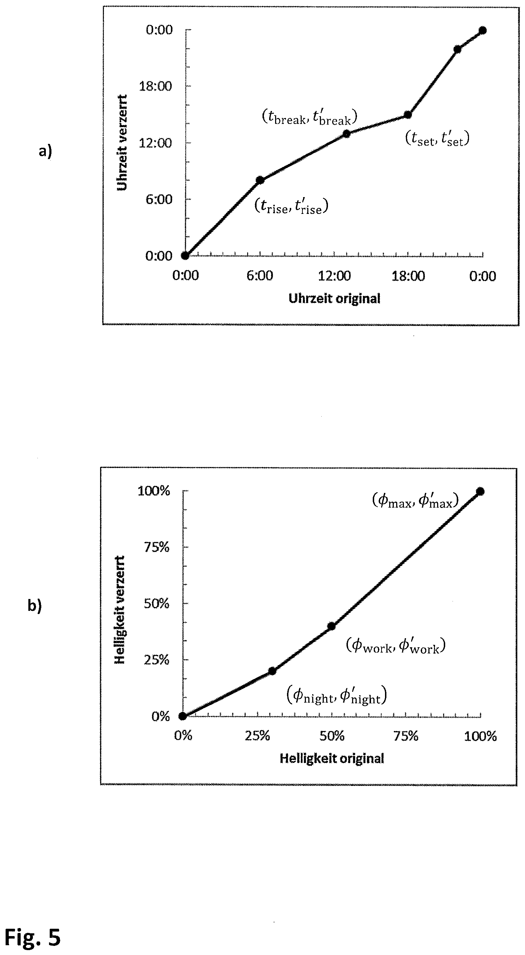

Die Ermittlung des Tagesverlaufs der Helligkeit φ am Tag d_j wird durchgeführt, indem sowohl die Zeitachse als auch die Werteachse für die Helligkeit unabhängig voneinander transformiert werden. Zur Transformation der Zeit werden die angegebenen Basiszeiten des vorbestimmten Tages d_b, zu welchem der Tagesverlauf H1 der Helligkeit vorliegt und gegen die Basiszeiten des Tages, zu welchem der Tagesverlauf der Helligkeit bestimmt werden soll, gegeneinander aufgetragen bzw. zugeordnet, siehe Figur 5a. Dabei sind die Basiszeiten des Tages, zu welchem der Tagesverlauf der Helligkeit bestimmt werden soll, gestrichen gekennzeichnet sowie die zugeordnete Y-Achse als "Uhrzeit verzerrt" angegeben. In der beschriebenen Ausführungsform liegt der Kurvenverlauf der Figur 3 in Form einer Vielzahl von Stützstellen auf, insbesondere auch Stützstellen, die bezüglich der Zeiten zwischen Basiszeiten liegen. Diese Zwischenzeiten werden gemäß der Darstellung der Figur 5a in der beschriebenen Ausführungsform durch lineare Interpolation ermittelt.

In gleicher Weise werden die Basiswerte der Helligkeit für beide Tage gegeneinander aufgetragen, wobei in der Zeichnung wiederum die Werte der Ausgangskurve ungestrichen und die Werte der gesuchten Kurven gestrichen gekennzeichnet sind. Zwischenwerte der Helligkeit für die Transformation sind auch hier durch lineare Interpolation zwischen zwei Basiswertpaaren durchführbar. Die Figur 5a stellt damit eine stückweise lineare Funktion f(t) dar, über welche die Zeiten des Tagesverlaufs am Tag d_b in Zeitpunkte am Tag d_j transformiert werden können. In gleicher Weise stellt Figur 5b eine Funktion g(φ) dar, mit welcher die Helligkeitswerte ausgehend vom Tagesverlauf am Tag d_b in die Helligkeitswerte am Tag d_j transformiert werden können, wobei in der beschriebenen Ausführungsform zwischen den Basiswertepaaren, beispielsweise (φ'_night, φ_night), (φ'_work, φ_work) eine lineare Interpolation zur Ermittlung von Zwischenwerten durchgeführt werden kann. In der Darstellung der Fig. 5b sind die Basiswerte der Helligkeit am Tag d_j gestrichen gekennzeichnet sowie die zugeordnete Y-Achse als "Helligkeit verzerrt" bezeichnet.

Auf der Grundlage der beschriebenen Transformation bzw. Abbildung der Zeitpunkte innerhalb eines Tages und der Helligkeitswerte kann nun der vorgegebene Tagesverlauf der Helligkeit gemäß Figur 3, der durch eine Vielzahl von in der Kurve nicht dargestellten Stützstellen {t_i, φ_i} in den gesuchten Tagesverlauf am Tag d_j ermittelt werden. Hierzu werden die Stützstellen {t_i, φ_i} abgebildet auf die Stützstellen {t'_n, φ'_n} mit stückweise linearen Funktionen t'_n = f(t_n) und φ'_n = g(φ_n).

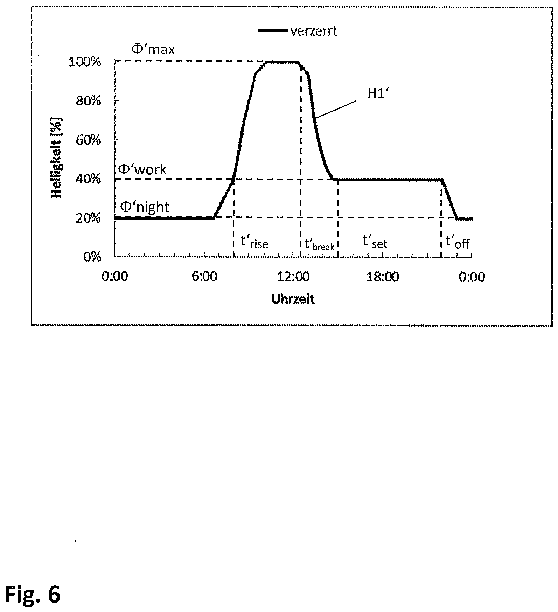

Figur 6 zeigt den transformierten Tagesverlauf H1`, der sich durch die ermittelten Stützstellen {t'_n, φ'_n} ergibt, unter Angabe der Basiszeiten t'_rise, t'_break, t'_set und t'_off sowie den Basiswerten für die Helligkeit φ'_night, φ'_work und φ'_max. In der Darstellung des Tagesverlauf H1` der Fig. 6 ist die zugeordnete Y-Achse wiederum als "Helligkeit verzerrt" bezeichnet.

Der Tagesverlauf F1 der in Figur 2 angegebenen Farbtemperatur kann in gleicher Weise wie der Verlauf H1 der Helligkeit transformiert werden, sodass die zentrale Steuereinrichtung beide transformierten Beleuchtungskurven H1, F1 des jeweiligen Tages d_j ermitteln kann, um entsprechende Steuersignale an die Betriebsgeräte 2 zu übermitteln zum Betrieb der LED-Einrichtungen 3, 3' zur Erzeugung des gewünschten Lichtprofils.

In einer weiteren Ausführungsform kann auch vorgesehen sein, weitere Beleuchtungsparameter wie beispielsweise eine Einstrahlrichtung oder die Größe einer Beleuchtungsfläche im Zeitverlauf variabel einzustellen, sodass auch ein solcher Beleuchtungsparameter wie für die Farbtemperatur und die Helligkeit beschrieben für einen vorbestimmten Tag im Tagesverlauf angegeben und für andere Tage berechnet werden kann. Hierzu kann das Beleuchtungssystem eingerichtet sein, steuerbare Lichtformeinrichtungen, beispielsweise bewegbare Spiegel und/oder Linseneinrichtungen anzusteuern, oder verschiedene Kanäle wie z.B. Direkt- und Indirektanteil einer Leuchte unabhängig voneinander zu dimmen.

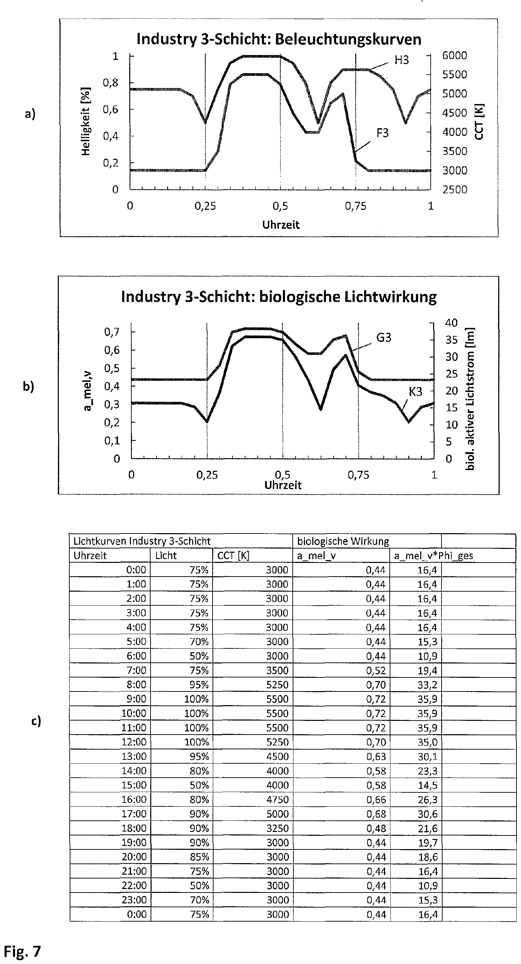

Wie erläutert, kann das erfindungsgemäße Beleuchtungssystem eingerichtet sein, für eine Vielzahl von Anwendungen entsprechende Tagesverläufe von Beleuchtungsparametern an einem vorbestimmten Tag sowie für eine Mehrzahl von Tagen innerhalb eines Jahreszyklus jeweils eine Mehrzahl von Basiszeiten und Basiswerten bereitzuhalten, um ausgehend von dem einen vorgegebenen Tagesverlauf für jeden Tag einen entsprechenden, in der vorliegenden Anmeldung als verzerrt bezeichneten Tagesverlauf zu ermitteln und auf der Grundlage dessen das Beleuchtungssystem zu steuern. In den Figuren 7 - 12 sind die zum beschriebenen Zweck optimierten und jeweils erfindungsgemäßen Verläufe der Helligkeit und der Farbtemperatur für jeweils einen dieser Anwendungsfälle angegeben, namentlich
- Figur 7a - c: für einen 3-Schichtbetrieb in einer Fabrikanlage,

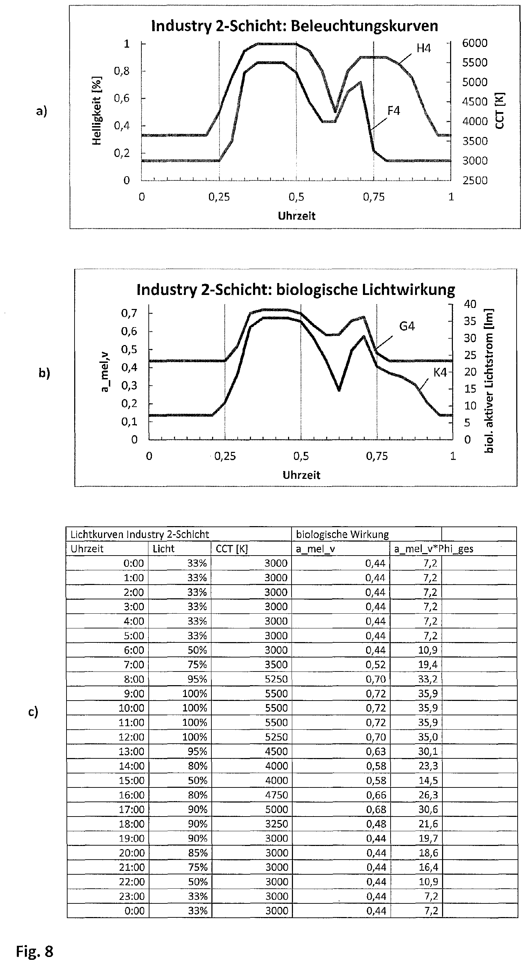

- Figur 8a - c: für einen 2-Schichtbetrieb in einer Fabrikanlage,

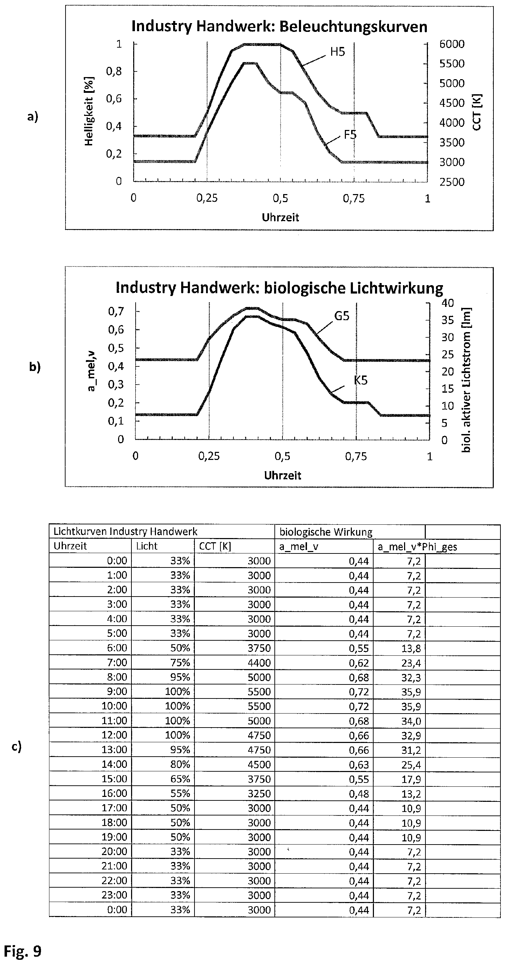

- Figur 9a - c: für die Beleuchtung in einem Handwerksbetrieb,

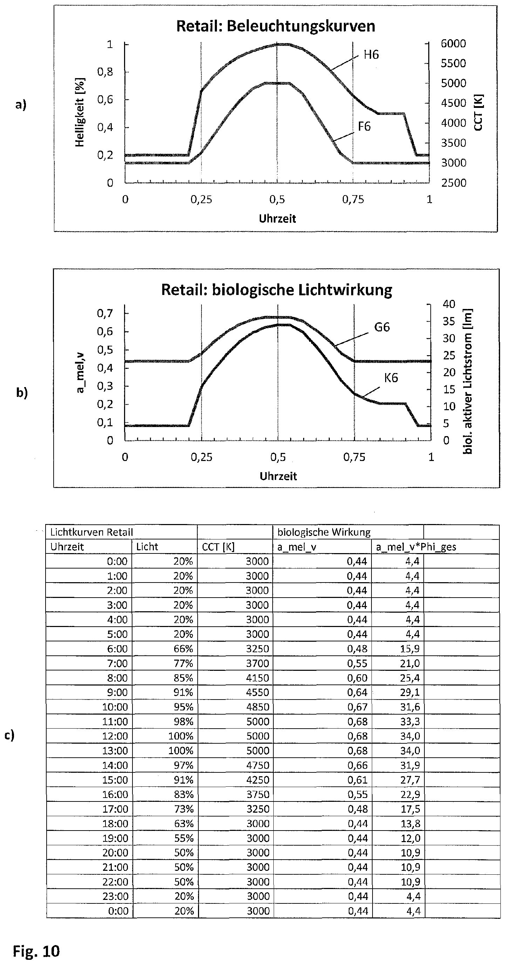

- Figur 10a - c: die Tagesverläufe für die Beleuchtungsparameter Helligkeit und Farbtemperatur für den Einzelhandel,

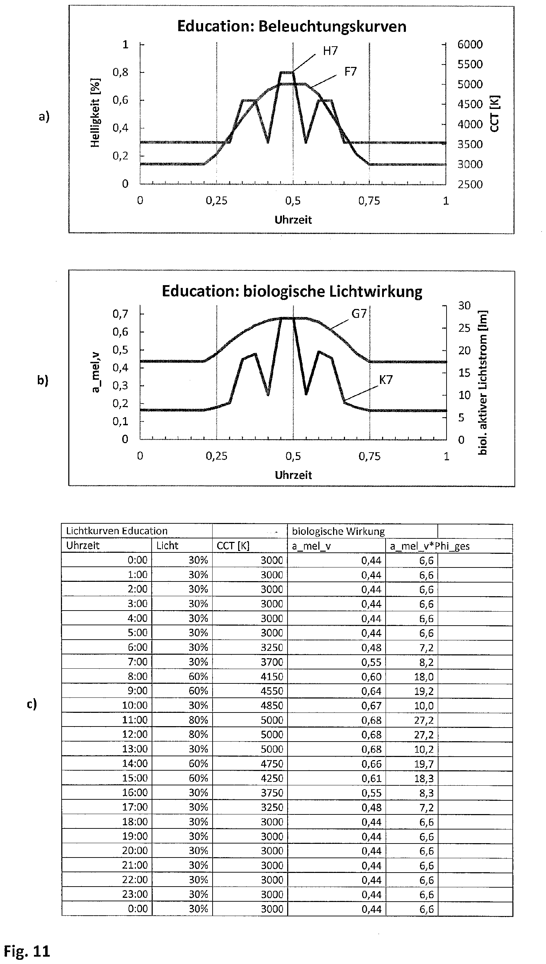

- Figur 11a - c: die Verläufe für einen Lernbetrieb wie eine Schule und

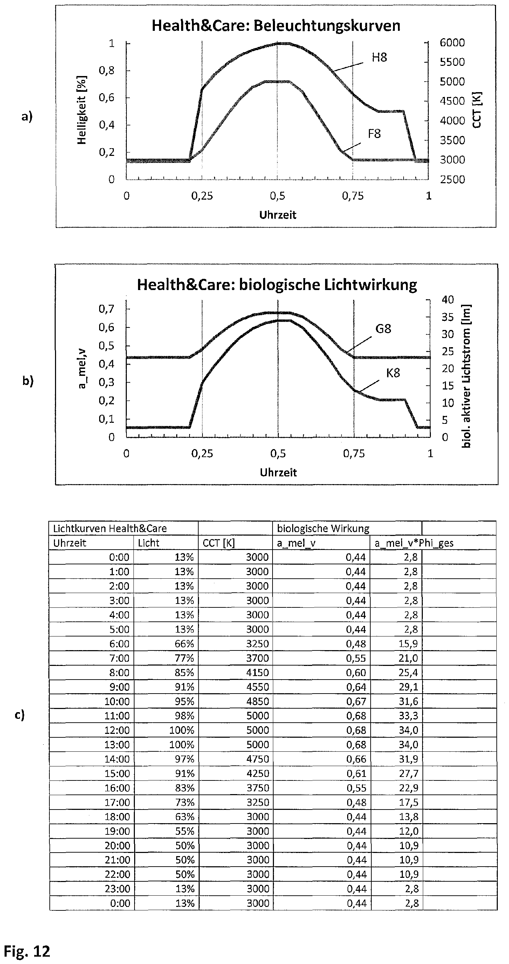

- Figur 12a - c: die Beleuchtungskurven für einen medizinischen Betrieb.

Wie der Fachmann erkennt, können in all diesen Anwendungsfällen, ausgehend von den vorgegebenen, optimierten und jeweils für sich erfindungsrelevanten Tagesverläufen an einem vorbestimmten Tag die jeweiligen optimalen Tagesverläufe an anderen Tagen ermittelt und das Beleuchtungssystem entsprechend zur Abgabe der jeweiligen Beleuchtungscharakteristik bzw. zur Abgabe des jeweiligen Lichtprofils angesteuert werden. Dabei hat sich herausgestellt, dass die angegebenen Tagesverlaufskurven auch abschnittsweise, d.h. über ein Zeitintervall kleiner 24 Stunden vorteilhaft und für sich erfindungsrelevant sein können.

Zu der Darstellung der Figur 7a, welche einen vorteilhaften, erfindungsgemäßen Verlauf von Helligkeit und Farbtemperatur für einen industriellen 3-Schichtbetrieb zeigt, sei noch darauf hingewiesen, dass es auch zweckmäßig sein kann, die Helligkeit (Kurve H3) so einzustellen, dass sie zu Beginn und während der dritten Schicht nicht mehr ansteigt, sondern auf dem niedrigen Niveau zum Ende der zweiten Schicht verharrt und damit konstant gehalten wird. In dieser Ausführungsform verlaufen Helligkeit und Farbtemperatur wie in Fig. 7a angegeben mit Ausnahme des Umstandes, dass die Helligkeit zwischen 22:00 und 6:00 auf 50% verbleibt.

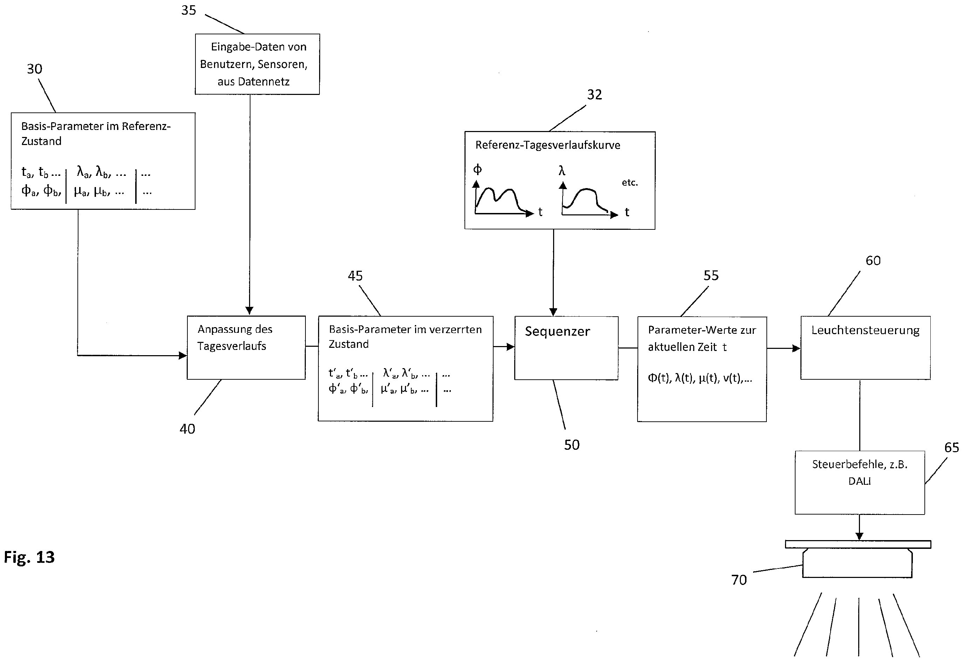

Mit Bezug auf die Figuren 13 - 19 soll das erfindungsgemäße Verfahren zur Einstellung eines Lichtprofils in einem Beleuchtungssystem für solche Fälle beschrieben werden, in welchen eine Mehrzahl von Einflussgrößen wie beispielsweise der Installationsort des Beleuchtungssystems, aktuelle Wetterdaten am Installationsort, die erfasste Nutzungssituation eines Raumes im Wirkbereich des Beleuchtungssystems und in Abhängigkeit der Altersstruktur der sich im Raum befindlichen Personen für die Festlegung einer Mehrzahl von Basiszeiten und/oder Mehrzahl von Basiswerten des helligkeitsrelevanten Beleuchtungsparameters und/oder die Mehrzahl von Basiswerten zumindest eines spektrumrelevanten Beleuchtungsparameters für einen Tag bzw. einen vorgegebenen Zeitraum von beispielsweise einigen Stunden herangezogen werden. Auf diese Weise ist das erfindungsgemäße Verfahren in der Lage, auf aktuelle Betriebssituationen zu reagieren, um das Lichtprofil des Beleuchtungssystems darauf anzupassen.

Figur 13 zeigt eine derartige Situation in einem Ablaufdiagramm, wobei das Verfahren von einem Basis-Parametersatz im Referenz-Zustand ausgeht. Vorgegeben sind Basiszeiten *tₐ,t_{b},*... sowie den diesen Basiszeiten zugeordnete Basis-Parameterwerte eines helligkeitsrelevanten Parameters *φₐ,φ_{b},*...; sowie mehrere spektrumrelevante Parameter *λₐ,λ_{b},*...; *µₐ,µ_{b},*...; *vₐ,v_{b},*..., welche sich aus den jeweiligen zeitlichen Referenzverläufen der genannten Parameter ergeben. Derartige spektrumrelevante Parameter ... können beispielsweise ausgewählt sein aus der Gruppe Farbtemperatur, Farbort, melanopischen Wirkfaktor und/oder weitere biologische Wirkfaktoren oder auch Lichtintensitäten in gewissen Spektrallinienbändern, wie beispielsweise die Intensität zwischen 380 und 385 nm oder die Lichtintensität zwischen 385 und 390 nm.

Zu jedem der Parameter ... wird eine Referenz-Kurve über den Tagesverlauf vorgegeben, wobei zur Verzerrung der Referenz-Kurven Basis-Zeiten *tₐ,t_{b},*... und Basis-Parameterwerte *φₐ,φ_{b},*...; *λₐ,λ_{b},*...; *µₐ,µ_{b},*...; *vₐ,v_{b},*... im Referenzzustand festgelegt werden. Die Verzerrung der Referenz-Kurven ist gemäß dem obenstehend beschriebenen Algorithmus dann durch die Angabe bzw. Berechnung der verzerrten Basis-Zeiten *t'ₐ,t'_{b},*... und der verzerrten Basis-Parameterwerte *φ'ₐ,φ'_{b},*...; *λ'ₐ,λ'_{b,}*...; *µ'ₐ,µ'_{b},*...; *v'ₐ,v'_{b},*... definiert. Wie in Figur 13 angegeben, erfolgt die Ermittlung der verzerrten Basis-Werte im Ansprechen auf eine Vielzahl von Einflussgrößen bzw. Eingabe-Daten von Benutzern, Sensoren oder dem Internet. Im Sequenzer erfolgt dann ausgehend von den Referenz-Tagesverlaufskurven für ... mit der Zuordnung von Referenz-Basiszeiten und zugehörigen Referenz-Basiswerten mit den verzerrten Basiszeiten und Basiswerten sowie der Interpolation für zwischenliegende Werte die Ermittlung der jeweiligen Parameter-Werte φ(t), λ(t), µ(t), ν(t)... zur aktuellen Zeit t. Auf der Grundlage dieser ermittelten helligkeits- und spektrumrelevanten Parameter-Werte werden in der Leuchtensteuerung dann die Betriebsparameter zur Einstellung des jeweiligen Lichtprofils ermittelt, in konkrete Steuerbefehle für die Leuchte(n), z.B. DALI-Dimmwerte für die verschiedenen ansteuerbaren Kanäle einer Leuchte, umgesetzt und an die Betriebsgeräte des Beleuchtungssystems übermittelt. Die in Figur 13 mit den Bezugszeichen 40,50 und 60 gekennzeichneten Schritte können beispielsweise in einer Steuereinrichtung, insbesondere einer Steuereinrichtung eines Betriebsgerätes durchgeführt werden.

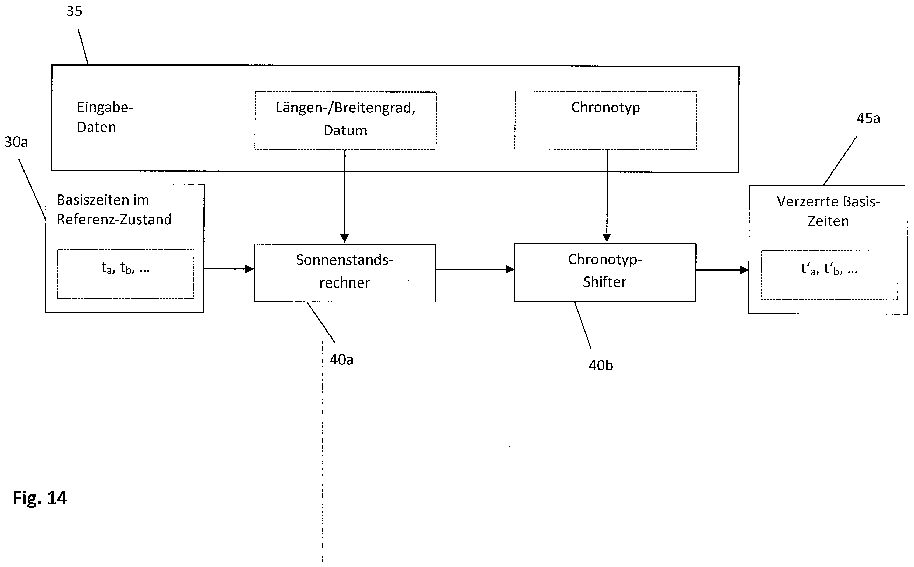

Punkt 40 des Ablaufdiagramms der Figur 13, d.h. die Anpassung des Tagesverlaufs ausgehend von den Referenz-Tagesverlaufskurven, abhängig von den möglichen Eingabedaten bzw. Einflussgrößen, kann in vielen Fällen unabhängig voneinander erfolgen, d.h. kann die Verzerrung der Basisparameter jeweils unabhängig von der eines anderen Basisparameters betrachtet werden. Beispielsweise kann häufig davon ausgegangen werden, dass die Basiszeiten *tₐ,t_{b},*...für sich verzerrt werden, ohne dass die Verzerrung der anderen Basisparameter *φₐ,φ_{b},*...; *λₐ,λ_{b},*...; *µₐ,µ_{b},*...; *vₐ,v_{b},*... hierzu berücksichtigt werden muss. Ein solches Beispiel zeigt Figur 14 für die Ermittlung der verzerrten Basiszeiten *t'ₐ,t'_{b},*... , ausgehend von den Basiszeiten im Referenz-Zustand *tₐ,t_{b},*.... Als Eingabedaten 35 dienen hier Längen-/Breitengrad des Installationsortes des Beleuchtungssystems, der beispielsweise in einer Steuereinrichtung des Beleuchtungssystems hinterlegt sein kann, sowie das aktuelle Datum für einen Sonnenstandsrechner 40a, welcher eine erste oder Vorverzerrung der Basiszeiten im Referenz-Zustand *tₐ,t_{b},*... festlegt. In dem angegebenen Beispiel wird als Eingangsdatum ferner der Chronotyp der sich im Wirkbereich des Beleuchtungssystems befindlichen Person bzw. Personen herangezogen und einem Chronotypshifter 40b weitergegeben, welcher die vom Sonnenstandsrechner 40a vorverzerrten Basiszeiten als Eingangsgröße erhält und diese um einen gewissen chronotypabhängigen Offset verschiebt.

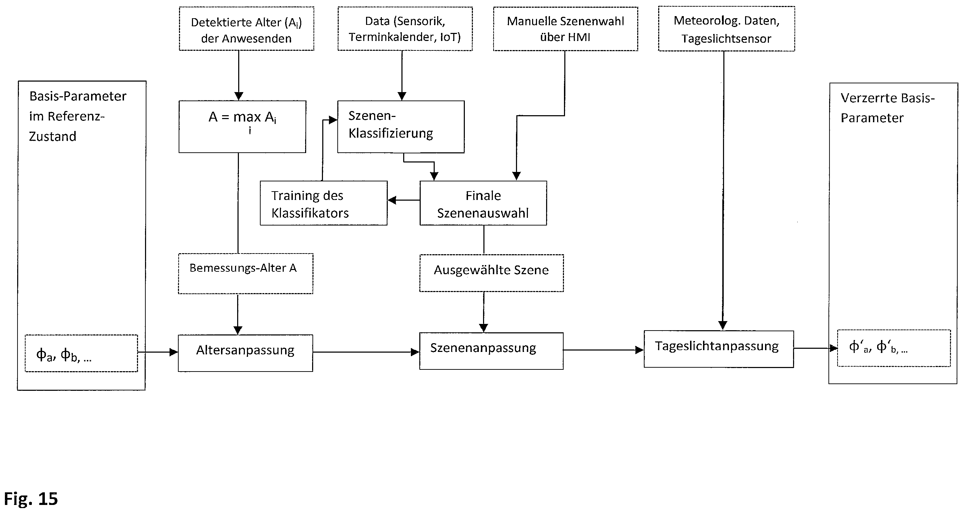

Figur 15 zeigt die Anpassung bzw. Verzerrung des helligkeitsrelevanten Parameters φ in Abhängigkeit einer Vielzahl von Einflussgrößen bzw. Eingabeparameter. In dem angegebenen Beispiel betrifft die erste Anpassung die Berücksichtigung des höheren Lichtbedarfs älterer Menschen. Hierzu betreffende Eingabedaten sind die Altersangaben aller im Wirkbereich des Beleuchtungssystems anwesenden Personen, beispielsweise aller in einem Raum sich befindlichen Personen.

Diese Mehrzahl von Altersangaben Aᵢ muss bei einer raumbezogenen Beleuchtung zunächst in Schritt 40c1 auf ein einziges Alter A reduziert werden. In dem in Figur 15 angegebenen Beispiel wird das Maximum der Altersangaben gebildet, da die älteste Person den höchsten Lichtbedarf hat, und jüngere Personen mit erhöhter Helligkeit in der Regel keine Probleme haben. Auf der Grundlage des ermittelten Bemessungsalters A kann dann in Schritt 40c eine erste Anpassung oder vor Verzerrung der Basiswerte durchgeführt werden, im beschriebenen Beispiel durch einen vom Bemessungsalter A abhängigen Erhöhungsfaktor β(A) größer eins, mit welchem die Referenz-Basiswerte des helligkeitsrelevanten Beleuchtungsparameters *φₐ,φ_{b},*...;multipliziert werden, um die angepassten bzw. verzerrten helligkeitsrelevanten Beleuchtungsparameter zu erhalten. Dieser Erhöhungsfaktor β(A) kann je nach Ausführungsform linear, exponentiell oder auch logarithmisch vom Bemessungsalter A abhängen. In der beschriebenen Ausführungsform wird der Erhöhungsfaktor gleich eins gesetzt bei einem Alter A_min, beispielsweise 25 Jahren, und ist dann konstant für niedrigere Werte des Alters.

Die zweite Anpassung in Schritt 40d ist eine Szenen-Anpassung, welche auf eine (quasi-)statische Szene stellen kann. Eine solche Szene kann beispielsweise eine Präsentation, ein Meeting, oder eine Bildschirmarbeit sein. Zu einer automatischen Szenenerkennung werden möglichst viele Eingangsdaten ("Data") 35 durch einen Klassifizierungs-Algorithmus 40d1 ausgewertet. In der Regel ist zusätzlich eine manuelle Szenen-Auswahl über ein Human-Machine-Interface (HMI) vorgesehen, um Fehler in der automatischen Erkennung zu kompensieren. In der beschriebenen Ausführungsform sollte daher die manuell gewählte Szene Vorrang erhalten. Die ausgewählte Szene wie eine Beamer-Präsentation wird der Szenen-Anpassung 40d übergeben, welche aus den damit verknüpften Daten, die im einer Steuereinrichtung abgelegt sind, die Basiswerte des helligkeitsrelevanten Beleuchtungsparameterparameter verändert. In der in Figur 15 angegebenen Ausführungsform wird der Klassifikationsalgorithmus 40d1 "trainiert", was in der Figur mit dem Bezugszeichen 40d2 angegeben ist. Hierzu wird ein Datensatz herangezogen, welcher für viele Fälle von Eingabedaten die korrekte Szene angibt. Eine falsch erkannte Szene führt zu einer manuellen Korrektur durch den Benutzer. Im laufenden Betrieb kann das System insofern "nachtrainiert" werden, indem diese manuellen Eingriffe in die Szenensteuerung dem Trainings-Datensatz hinzugefügt werden.

Im letzten Schritt 40e wird eine Tageslichtanpassung durchgeführt, welche auf meteorologischen Daten oder Tageslichtsensordaten basiert. Hier wird das natürliche einfallende Licht berücksichtigt, um den Anteil des künstlichen Lichts zu reduzieren und in der Gesamtsumme die gewünschte Lichtmenge zu erhalten. Als Resultat der Abläufe gemäß Figur 15 werden, ausgehend von den Basisparametern *φₐ,φ_{b},*...; 30b im Referenzzustand die verzerrten Basisparameter *φₐ,φ_{b},*...; ausgegeben, auf der Grundlage dessen dann die Parameter-Werte 55 zur aktuellen Zeit t ermittelt werden, siehe Figur 13.

In einer Ausführungsform kann eine Altersanpassung des helligkeitsrelevanten Beleuchtungsparameters so ausgebildet sein, dass sich beispielsweise der Parameterwert φₐ(A) ergibt zu *φₐ*(*A*)=*αₐ*(*A*)·*φₐ*(*A*ₘₐₓ) mit *αₐ*(*A*)∈[*αₘᵢₙ*,1] und *αₘᵢₙ*>0.

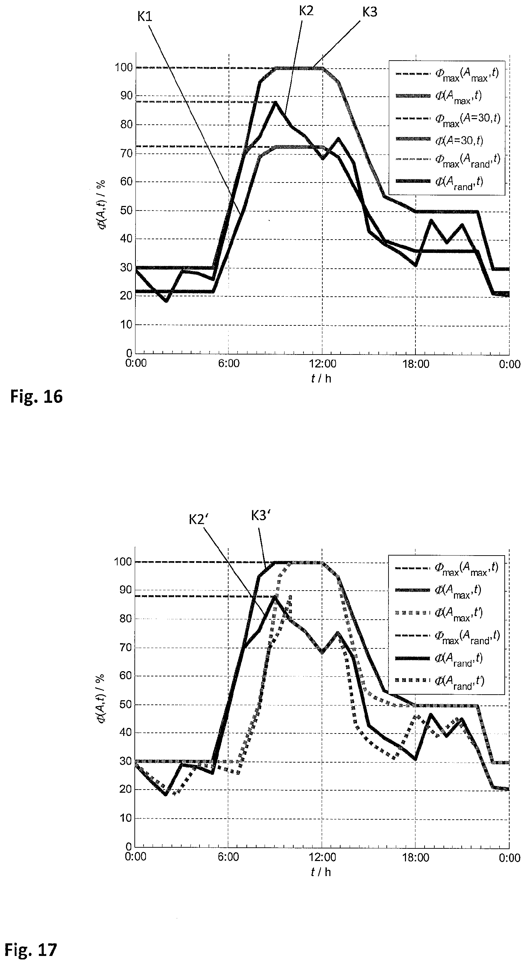

Dabei stellt *A*ₘₐₓ hier das hinterlegte maximale Alter, z. B. Aₘₐₓ=100 Jahre dar und nicht das erkannte Bemessungsalter *A*=maxᵢ*A*ᵢ. Neben *αₐ*(*A*) können ebenso *α_{b}*(*A*),... definiert werden, die von *αₐ*(*A*) abweichen können. Dabei können die Funktionen *αₐ*(*A*), *α_{b}*(*A*), ... je nach Ausführungsform insbesondere eine konstante, lineare oder nichtlineare Abhängigkeit angesetzt werden. Figur 16 zeigt einen solchen Tagesverlauf des helligkeitsrelevanten Beleuchtungsparameters bzw. der Helligkeit unter Berücksichtigung der Eigenschaft Alter, wobei eine lineare Abhängigkeit des Parameters α vom Alter A angesetzt wurde. Im dargestellten Beispiel sind die Helligkeiten, d.h. die Helligkeit relevanten Beleuchtungsparameter für ein statisches Alter, z.B. 30 Jahre (Kurve K1), sowie für ein zufallsverteiltes, mittleres Alter (Kurve K2) dargestellt. Die Kurve K3 stellt den Tagesverlauf für die maximale Helligkeit φ(Aₘₐₓ) dar, ohne dass weitere Eingabedaten wie Szenen berücksichtigt wurden.

In Kombination mit der Verzerrung der Zeitachse mit *t*ᵣᵢₛₑ→*t'*ᵣᵢₛₑ und *t*ₛₑₜ→*t*'ₛₑₜ bzw. t_a -> t'_a und **t_set -> t'_set** ergibt sich ein beispielhafter Verlauf wie in Figur 17 angegeben. Dabei ist die Verzerrungskurve K3` der Maximalkurve K3 und die Verzerrungskurve K2' der zufallsverteilte Kurve K2 der Figur 16 dargestellt. Zur Vereinfachung der Darstellung ist nur die Eigenschaft A (Alter) zur Anpassung des Tagesverlaufs helligkeitsrelevanten Beleuchtungsparameters und die Verzerrung der Basiszeiten wie mit Bezug auf Figur 14 beschrieben berücksichtigt.

In einer weiteren Ausführungsform kann α nicht nur von einer Eigenschaft wie dem Alter, sondern von mehreren Eigenschaften abhängen, sodass sich ein Zusammenhang α(A, B, C...) ergibt. Dadurch kann zusätzlich zu einem dynamischen Tagesverlauf eine weitere Dynamik durch diese Eigenschaften in die Helligkeit *Φ*, Farbtemperatur oder das Spektrum einfließen. Durch gewichtete Faktoren von Eigenschaften kann bestimmt werden, ob und welche Eigenschaften Einfluss auf das Lichtprofil haben. Darüber hinaus können sich auch mehrere Faktoren *α*(*A,B*,*C*,...),*β*(*D*,*E*,*F*,...),*γ*(*H*,*J*,K,...),... ergeben, die zu einem Vorfaktor verrechnet werden.

### Bezugszeichenliste

- 1: Beleuchtungssystem
- 2: Betriebsgerät
- 3, 3': LED-Einrichtung
- 4: Zentrale Steuereinrichtung
- 5: DALI-Bus
- 6: Netzwerkschnittstelle
- 7: Netzwerk
- 8: Netzwerkspeicher
- 9: Server
- 20: DALI-Schnittstelle
- 24: Lokale Steuereinrichtung
- 25: Speicher
- 30: Basis-Parameter im Referenz-Zustand
- 30a: Basiszeiten im Referenz-Zustand
- 30b: Basis-Parameter im Referenz-Zustand
- 32: Referenz-Tagesverlaufskurven
- 35: Eingabe-Daten von Benutzern, Sensoren, aus einem Datennetz
- 40: Anpassung des Tagesverlaufs
- 40a: Sonnenstandsrechner
- 40b: Chronotyp-Stifter
- 40c: Altersanpassung
- 40c1: Bestimmung des Bemessungsalters A
- 40d: Szenenanpassung
- 40d1: Szenen-Klassifizierung
- 40d2: Training des Klassifikation
- 40e: Tageslichtanpassung
- 45: Basis-Parameter im verzerrten Zustand
- 45a: verzerrte Basis-Zeiten
- 45b: verzerrte Basis-Parameter
- 50: Sequenzer
- 55: Parameter-Werte zur aktuellen Zeit t
- 60: Leuchtensteuerung
- 65: Steuerbefehle
- 70: Leuchte

## Patentansprüche

1. Verfahren zur Einstellung eines Lichtprofils in einem Beleuchtungssystem, umfassend die Schritte:
- Bereitstellen einer mittels Stützstellen angegebener Referenzkurve eines Tagesverlaufs eines helligkeitsrelevanten Beleuchtungsparameters φ(t),
wobei die Stützstellen eine Mehrzahl von Basiszeiten (t_rise_b, t_set_b, t_off_b; t_a_b, t_b_b; t_c_b) und eine Mehrzahl von den Basiszeiten zugeordneten Basiswerten (φ_max_b, φ_night_b,
φ_work_b; φ_a_b, φ_b_b, Φ_c_b) des helligkeitsrelevanten Beleuchtungsparameters umfassen, und einer mittels Stützstellen angegebener Referenzkurve eines Tagesverlaufs eines spektrumrelevanten Beleuchtungsparameters λ(t),
wobei die Stützstellen die Mehrzahl von Basiszeiten (t_rise_b, t_set_b, t_off_b; t_a_b, t_b_b; t_c_b) und eine Mehrzahl von den Basiszeiten zugeordneten Basiswerten (λ _max_b, λ _night_b, λ_work_b; λ _a_b, λ _b_b, λ_c_b) des einen spektrumrelevanten Beleuchtungsparameters umfassen, jeweils für einen vorbestimmten Tag (d_b);
- Bereitstellen einer korrespondierenden Mehrzahl von Basiszeiten (t_rise_j, t_set_j, t_off_j; t_a_j, t_b_j; t_c_j), einer Mehrzahl von den Basiszeiten zugeordneten Basiswerten (φ_max_j, φ_night_j, φ_work_j; φ_a_j, φ_b_j, φ_c_j) des helligkeitsrelevanten Beleuchtungsparameters und einer Mehrzahl von den Basiszeiten zugeordneten Basiswerten (λ _max_j, λ _night_j, λ_work_j; λ _a_j, λ _b_j, λ_c_j) des einen spektrumrelevanten Beleuchtungsparameters, jeweils für zumindest einen anderen Tag (d_j);
- Ermitteln eines mittels Stützstellen angegebenen Tagesverlaufs des helligkeitsrelevanten Beleuchtungsparameters φ(t) und eines mittels Stützstellen angegebenen Tagesverlaufs des einen spektrumrelevanten Beleuchtungsparameters X(t) für jeden zumindest einen anderen Tag (d_j),
wobei die jeweiligen Basiszeiten des vorbestimmten Tages (d_b) und jeweilige Basiszeiten des anderen Tages (d_j), die jeweiligen Basiswerte des helligkeitsrelevanten Beleuchtungsparameters des vorbestimmten Tages (d_b) und jeweilige Basiswerte des helligkeitsrelevanten Beleuchtungsparameters des anderen Tages (d_j) sowie die jeweiligen Basiswerte des einen spektrumrelevanten Beleuchtungsparameters des vorbestimmten Tages (d_b) und jeweilige Basiswerte des einen spektrumrelevanten Beleuchtungsparameters des anderen Tages (d_j) einander zugeordnet werden und zwischenliegende Zeiten und zwischenliegende Werte interpoliert, insbesondere linear interpoliert werden, sodass die jeweiligen Basiszeiten bzw. die jeweiligen Basiswerte des vorbestimmten Tages und des jeweils anderen Tages achsenweise abgebildet werden, und sich der jeweilige Tagesverlauf des anderen Tages (d_j) durch abschnittsweises Stauchen, Strecken oder Verschieben der entsprechenden Referenzkurve des Tagesverlaufs des vorbestimmten Tages ergibt, und
- Steuern bzw. Regeln von Betriebsparametern des Beleuchtungssystems zur Einstellung des ermittelten Tagesverlaufs des helligkeitsrelevanten Beleuchtungsparameters φ'(t') und des einen spektrumrelevanten Beleuchtungsparameters λ'(t') für jeden zumindest einen anderen Tag (d_j).

2. Verfahren nach Anspruch 1, **dadurch gekennzeichnet, dass** ferner die Schritte umfasst sind:
- Bereitstellen einer mittels Stützstellen angegebener Referenzkurve eines Tagesverlaufs zumindest eines weiteren spektrumrelevanten Beleuchtungsparameters (µ(t), .... , v(t)), insbesondere zum Einstellen eines biologischen Wirkfaktors wie eines melanopischen Wirkfaktors für den vorbestimmten Tag (d_b),
wobei die Stützstellen eine Mehrzahl von den Basiszeiten für den vorbestimmten Tag (d_b) zugeordneten Basiswerten des weiteren spektrumrelevanten Beleuchtungsparameters (µ(t), .... , ν(t)) (φ_max_b, φ_night_b, φ_work_b; φ_a_b, φ_b_b, φ_c_b) umfassen;
- Bereitstellen einer korrespondierenden Mehrzahl von den Basiszeiten des zumindest einen anderen Tages (d_j) zugeordneten Basiswerten (µ _max_j, µ _night_j, µ_work_j, ... , ν_max_j, ν_night_j, ν_work_j; (µ _a_j, µ b_j, p_c_j, ... , v_a_j, v_b_j, v_c_j) des weiteren spektrumrelevanten Beleuchtungsparameters für den zumindest einen anderen Tag (d_j);
- Ermitteln eines mittels Stützstellen angegebenen Tagesverlaufs des weiteren spektrumrelevanten Beleuchtungsparameters (µ(t), .... , ν(t)) für jeden zumindest einen anderen Tag (d_j),
wobei die jeweiligen Basiswerte des weiteren spektrumrelevanten Beleuchtungsparameters des vorbestimmten Tages (d_b) und die jeweiligen Basiswerte des weiteren spektrumrelevanten Beleuchtungsparameters des anderen Tages (d_j) einander zugeordnet werden und zwischenliegende Werte interpoliert, insbesondere linear interpoliert werden, sodass die jeweiligen Basiswerte des weiteren spektrumrelevanten Beleuchtungsparameters des vorbestimmten Tages und die jeweiligen Basiswerte des weiteren spektrumrelevanten Beleuchtungsparameters des jeweils anderen Tages achsenweise abgebildet werden, und sich der jeweilige Tagesverlauf des anderen Tages (d_j) durch abschnittsweises Stauchen, Strecken oder Verschieben der entsprechenden Referenzkurve des jeweiligen Tagesverlaufs des vorbestimmten Tages ergibt, und
- Neben dem Steuern bzw. Regeln von Betriebsparametern des Beleuchtungssystems zur Einstellung des ermittelten Tagesverlaufs des helligkeitsrelevanten Beleuchtungsparameters φ'(t') und des einen spektrumrelevanten Beleuchtungsparameters λ'(t'), ferner Steuern bzw. Regeln von Betriebsparametern des Beleuchtungssystems zum Einstellen des ermittelten Tagesverlaufs des weiteren spektrumrelevanten Beleuchtungsparameters (µ'(t), .... , ν'(t)), jeweils für jeden zumindest einen anderen Tag (d_j) .

3. Verfahren nach einem der Ansprüche 1 und 2,
**dadurch gekennzeichnet, dass** die Mehrzahl von Basiszeiten und/oder die Mehrzahl von Basiswerten des helligkeitsrelevanten Beleuchtungsparameters und/oder die Mehrzahl von Basiswerten des zumindest einen spektrumrelevanten Beleuchtungsparameters in Abhängigkeit vom Installationsort des Beleuchtungssystems, jeweils für zumindest einen anderen Tag festgelegt werden.

4. Verfahren nach einem der Ansprüche 1 bis 3,
**dadurch gekennzeichnet, dass** eine Mehrzahl von nutzungsspezifischen Tagesverläufen des helligkeitsrelevanten Beleuchtungsparameters (φ(t)) und des zumindest einen spektrumrelevanten Beleuchtungsparameters (λ(t), µ(t), .... , ν(t))) für den vorbestimmten Tag (d_b) zur Auswahl bereitgestellt werden.

5. Verfahren nach einem der Ansprüche 1 bis 4,
**dadurch gekennzeichnet, dass** die Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen (d_j) in Abhängigkeit vom Installationsort des Beleuchtungssystems, in Abhängigkeit der Jahreszeit am Installationsort des Beleuchtungssystems und/oder in Abhängigkeit von meteorologischen und/oder klimatischen Daten, insbesondere Wetterdaten am Installationsort des Beleuchtungssystems für den zumindest einen anderen Tag (d_j) festgelegt werden.

6. Verfahren nach einem der Ansprüche 1 bis 5,
**dadurch gekennzeichnet, dass** die Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen (d_j) in Abhängigkeit von zumindest einer, sich im Wirkbereich des Beleuchtungssystems befindlichen Person festgelegt wird.

7. Verfahren nach Anspruch 6, **dadurch gekennzeichnet, dass** die Festlegung auf der Grundlage von objektiven Eigenschaften der zumindest einen Person, wie Alter, Geschlecht, Bewegung oder subjektiven Eigenschaften bzw. Empfinden der zumindest einen Person, wie Lichtpräferenz, Stimmung oder Chronotyp durchgeführt wird.

8. Verfahren nach einem der Ansprüche 6 oder 7,
**dadurch gekennzeichnet, dass** zumindest eine objektive oder subjektive Eigenschaft bzw. Empfinden der zumindest einen Person mittels einer Datenverarbeitung von über eine Sensoreinrichtung erfassten Daten, insbesondere Bilddaten, ermittelt wird.

9. Verfahren nach einem der Ansprüche 5 bis 8,
**dadurch gekennzeichnet, dass** die Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen (d_j) durch Benutzereingabe (Human-Machine-Interface), durch eine Datenverarbeitung von über eine Sensoreinrichtung und/oder über eine Netzwerkverbindung erfassten Daten festgelegt werden.

10. Verfahren nach einem der Ansprüche 1 bis 9,
**dadurch gekennzeichnet, dass** die Festlegung der Mehrzahl von Basiszeiten und/oder die Mehrzahl von Basiswerten des helligkeitsrelevanten Beleuchtungsparameters und/oder die Mehrzahl von Basiswerten zumindest eines spektrumrelevanten Beleuchtungsparameters für zumindest einen aus der Mehrzahl von über ein Jahr verteilten Tagen in Abhängigkeit einer Mehrzahl von Eingabedaten erfolgt.

11. Beleuchtungssystem, umfassend ein Betriebsgerät (2) zum Speisen zumindest eines Leuchtmittels, eine Steuereinrichtung (4) sowie einen Speicher (8) zum Speichern von vorgegebenen. Beleuchtungskurven, wobei die Steuereinrichtung eingerichtet und ausgebildet ist, gemäß einem Verfahren nach einem der Ansprüche 1 bis 10 die jeweiligen Tagesverläufe der Beleuchtungsparameter zu ermitteln und entsprechende Steuersignale an das Betriebsgerät (2) zu übermitteln zur Einstellung des Lichtprofils im Beleuchtungssystem.

## Claims

1. Method for adjusting a light profile in a lighting system comprising the steps:
- providing a reference curve indicated by means of support points of a day schedule of a brightness-related lighting parameter φ (t),
wherein the supports points comprise a multitude of basic times
(t_rise_b, t_set_b, t_off_b; t_a_b, t_b_b; t_c_b) and a multitude of basic values (φ_max_b, φ_night_b, φ_work_b; φ_a_b, φ_b_b, φ_c_b) assigned to the basic times of the brightness-related lighting parameter and a reference curve indicated by means of support points of a day schedule of a spectrum-related lighting parameter λ (t),
wherein the support points comprise the multitude of basic times
(t_rise_b, t_set_b, t_off_b; t_a_b, t_b_b; t_c_b) and a multitude of basic values (λ_max_b, λ_night_b,_work_b; λ_a_b, A_b_b, λ_c_b) assigned to the basic times of the spectrum-related lighting parameter respectively for a predefined day (d_b);
- providing a corresponding multitude of basic times (t_rise_j, t_set_j, t_off_j; t_a_j, t_b_j; t_c_j), of a multitude of basic values (φ_max_j, φ_night_j, φ_work_j; φ_a_j, φ_b_j, φ_c_j) assigned to the basic times of the brightness-related lighting parameter and of a multitude of basic values (λ_max_j, λ_night_j, λ_ work_j; λ_a_j, λ_b_j, λ_c_j) assigned to the basic times of the one spectrum-related lighting parameter, respectively for at least one other day (d_j);
- detection of a day schedule indicated by means of support points of the brightness-related lighting parameter φ (t) and of a day schedule indicated by means of support points of the spectrum-related lighting parameter λ (t) for each at least one other day (dJ),
wherein the respective basic times of the predefined day (d_b) and respective basic times of the other day (d_j), the respective basic values of the brightness-related lighting parameter of the predefined day (d_b) and respective basic values of the brightness-related lighting parameter of the other day (d_j) as well as the respective basic values of the one spectrum-related lighting parameter of the predefined day (d_b) and respective basic values of the one spectrum-related lighting parameter of the other day (d_j) are assigned to one another and intermediate times and intermediate values are interpolated, in particular linearly interpolated, so that the respective basic times or the respective basic values or the respective basic values of the predefined day and of the respectively other day are represented axis by axis and the respective day schedule of the other day (d_j) results by compressing, stretching or displacing in sections the corresponding reference curve of the day schedule of the predefined day and
- controlling or adjusting operating parameters of the lighting system for setting the detected day schedule of the brightness-related lighting parameter φ' (t') and of the one spectrum-related lighting parameter λ'(t') for each at least one other day (dJ).

2. Method according to claim 1, **characterized in that** it furthermore comprises the steps:
- providing of a reference curve indicated by means of support points of a day schedule of at least one further spectrum-related lighting parameter (µ(t), ..., *v*(t)), in particular for adjusting a factor of biological effectiveness such as a melanopic effect factor for the predefined day (d_b),
wherein the support points comprise a multitude of the basic values assigned to the basic times for the predefined day (d_b) of the further spectrum-related lighting parameter (µ(t), ..., *v*(t)) (φ_max_b, φ_night_b, φ_work_b; φ_a_b, φ_b_b, φ_c_b);
- providing of a corresponding multitude of basic values (µ_max_j, µ_night_j, µ_work_j, ..., *v*_axj, *ν*_night_j, *v*_workj; (µ_a_j, µ_b_j, µ_c_k, ..., v_aj, v_b_j, v_c_j) assigned to the basic times of the at least one other day (d_j) of the further spectrum-related lighting parameter for the at least one other day (d_j);
- detection of a day schedule indicated by means of support points of the further spectrum-related lighting parameter (µ(t), ..., *v*(t)) for each at least one other day (d_j),
wherein the respective basic times of the further spectrum-related lighting parameter of the predefined day (d_b) and the respective basic values of the further spectrum-related lighting parameter of the other day (d_j) are assigned to one another and intermediate values are interpolated, in particular linearly interpolated, so that the respective basic values of the further spectrum-related lighting parameter of the predefined day and the respective basic values of the further spectrum-related lighting parameter of the respective other day are represented axis by axis and the respective day schedule of the other day (d_j) results from compressing, stretching or displacing in sections the corresponding reference curve of the respective day schedule of the predefined day and
- besides controlling or adjusting operating parameters of the lighting system for setting the detected day schedule of the brightness-related lighting parameter φ' (t') and of the one spectrum-related lighting parameter λ'(t'), in addition controlling or adjusting operating parameters of the lighting system for setting the detected day schedule of the further spectrum-related lighting parameter (µ'(t), ..., v'(t)), respectively for each at least one other day (d_j).

3. Method according to one of the claims 1 and 2, **characterized in that** the multitude of basic times and/or the multitude of basic values of the brightness-related lighting parameter and/or the multitude of basic values of the at least one spectrum-related lighting parameter is set depending on the installation location of the lighting system, respectively for at least one other day.

4. Method according to one of the claims 1 to 3, **characterized in that** a multitude of use-specific day schedules of the brightness-related lighting parameter φ (t) and of the at least one spectrum-related lighting parameter (A (t), µ(t), ..., *v*(t))) is provided for the predefined day (d_b) for selection.

5. Method according to one of the claims 1 to 4, **characterized in that** the multitude of basic times and/or the multitude of basic values of the brightness-related lighting parameter and/or the multitude of basic values of at least one spectrum-related lighting parameter is set for at least one of the multitude of days (d_j) spread over a year depending on the installation location of the lighting system and/or depending on meteorological and/or climatic data, in particular weather data at the installation location of the lighting system for the at least one other day (d_j).

6. Method according to one of the claims 1 to 5, **characterized in that** the multitude of basic times and/or the multitude of basic values of the brightness-related lighting parameter and/or the multitude of basic values of at least one spectrum-related lighting parameter is set for at least one of the multitude of days (d_j) spread over a year depending on at least one person situated in the effective range of the lighting system.

7. Method according to claim 6, **characterized in that** the setting is realized on the base of objective characteristics of the at least one person such as age, sex, movement or subjective characteristics or perception of the at least one person such as light preference, ambiance or chronotype.

8. Method according to one of the claims 6 or 7, **characterized in that** at least one objective or subjective characteristic or the perception of the at least one person is detected by means of data treatment of data recorded by a sensor device, in particular image data.

9. Method according to one of the claims 5 to 8, **characterized in that** the multitude of basic times and/or the multitude of basic values of the brightness-related lighting parameter and/or the multitude of basic values of at least one spectrum-related lighting parameter is set for at least one of the multitude of days (d_j) spread over a year by user input (human-machine-interface), by data treatment of data recorded by a sensor device and/or overa network connection.

10. Method according to one of the claims 1 to 9, **characterized in that** the setting of the multitude of basic times and/or the multitude of basic values of the brightness-related lighting parameter and/or the multitude of basic values of at least one spectrum-related lighting parameter is set for at least one of the multitude of days (d_j) spread over a year depending on a multitude of input data.

11. Lighting system comprising a ballast (2) for feeding at least one lighting means, a control device (4) as well as a memory (8) for storing predefined lighting curves, wherein the control device is designed and configured to detect the respective day schedules of the lighting parameters according to a method according to one of the claims 1 to 10 and to transmit corresponding control signals to the ballast (2) for adjusting the light profile in the lighting system.

## Revendications

1. Procédé pour régler un profil lumineux dans un système d'éclairage comprenant les étapes :
- mise à disposition d'une courbe de référence, indiquée par des points d'appui, d'une routine quotidienne d'un paramètre d'éclairage pertinent pour la luminosité (φ) (t),
cependant que les points d'appui comprennent une multitude de temps de base (t_rise_b, t_set_b, t_off_b ; t_a_b, t_b_b ; t_c_b) et une multitude de valeurs de base (φ_max_b, φ_night_b, (φ) _work_b ; φ _a_b, φ _b_b, (φ) _c_b) associées aux temps de base du paramètre d'éclairage pertinent pour la luminosité,
et d'une courbe de référence, indiquée par des points d'appui, au cours d'une journée d'un paramètre d'éclairage pertinent pour le spectre λ (t), cependant que les points d'appui comprennent une multitude de temps de base (t_rise_b, t_set_b, t_off_b ; t_a_b, t_b_b ; t_c_b) et une multitude de valeurs de base (λ_max_b, λ _night_b, λ _work_b ; λ _a_b, λ _b_b, λ _c_b) associées aux temps de base du paramètre d'éclairage pertinent pour le spectre respectivement pour un jour prédéfini (d_b) ;
- mise à disposition d'une multitude correspondante de temps de base (t_rise_j, t_set_j, t_off_j ; t_a_j, t_b_j ; t_c_j), d'une multitude de valeurs de base (φ_max_j), φ_night_j, φ _work_j ; φ _a_j, φ _b_j, φ _c_j) associées aux temps de base du paramètre d'éclairage pertinent pour la luminosité et d'une multitude de valeurs de base (λ_max_j, λ _night_j, λ _ work_j ; λ_a_j, λ_b_j, À_cj) associées aux temps de base du paramètre d'éclairage pertinent pour le spectre, respectivement pour au moins un autre jour (d J) ;
- détection d'une routine quotidienne indiquée par des points d'appui du paramètre d'éclairage pertinent pour la luminosité φ (t) et d'une routine quotidienne indiquée par des points d'appui du paramètre d'éclairage pertinent pour le spectre λ (t) pour chaque au moins autre jour (d_j),
cependant que les temps de base respectifs du jour prédéfini (d_b) et des valeurs de base respectives de l'autre jour (d_j), les temps de base respectifs du paramètre d'éclairage pertinent pour la luminosité du jour prédéfini (d_b) et des valeurs de base respectives du paramètre d'éclairage pertinent pour la luminosité de l'autre jour (d_j) ainsi que les valeurs de base respectives du paramètre d'éclairage pertinent pour le spectre du jour prédéfini (d_b) et des valeurs de base respectives du paramètre d'éclairage pertinent pour le spectre de l'autre jour (dJ) sont associées les unes aux autres et des temps intermédiaires et des valeurs intermédiaires sont interpolées, en particulier sont interpolées linéairement, si bien que les temps de base respectifs ou les valeurs de base respectives du jour prédéfini et de l'autre jour respectif sont représentés par axe et que la routine quotidienne respective de l'autre jour (d_j) résulte de la compression, de l'étirement ou du déplacement par section de la courbe de référence correspondante de la routine quotidienne du jour prédéfini et
- commande ou réglage de paramètres de fonctionnement du système d'éclairage pour le réglage de la routine quotidienne détectée du paramètre d'éclairage pertinent pour la luminosité φ' (t') et du paramètre d'éclairage pertinent pour le spectre λ' (t') pour chaque au moins autre jour (d_j).

2. Procédé selon la revendication 1, **caractérisé en ce qu'**il comprend de plus les étapes :
- mise à disposition d'une courbe de référence, indiquée par des points d'appui, d'une routine quotidienne d'au moins un autre paramètre d'éclairage pertinent pour le spectre (µ(t), ..., ν(t), en particulier pour régler un facteur d'efficacité biologique comme un facteur d'efficacité mélanopique pour le jour prédéfini (d_b),
cependant que les points d'appui comprennent une multitude de valeurs de base, associées aux temps de base pour le jour prédéfini (d_b), de l'autre paramètre d'éclairage pertinent pour le spectre (µ(t), .... *v*(t)) (φ_max_b, φ_night_b, φ_work_b ; φ_a_b, φ_b_b, φ_c_b) ;
- mise à disposition d'une multitude correspondante de valeurs de base, associées aux temps de base de l'au moins un autre jour (d_j), (µ_max_j, µ_night_j, µ_work_j, ..., *ν*_ax_j, *ν*_night_j, *ν*_work_j ; (µ_a_j, µ_b_j, µ_c_k, ..., *ν*_a_j, *v*_b_j, *v*_c_j) de l'autre paramètre d'éclairage pertinent pour le spectre pour le au moins un autre jour (d_j),
- détection d'une routine quotidienne indiquée au moyen de points d'appui de l'autre paramètre d'éclairage pertinent pour le spectre (µ(t), ..., v(t)) pour chaque au moins un autre jour (dJ),
cependant que les valeurs de base respectives de l'autre paramètre d'éclairage pertinent pour le spectre du jour prédéfini (d_b) et les valeurs de base respectives de l'autre paramètre d'éclairage pertinent pour le spectre de l'autre jour (d_j) sont associées les unes aux autres et des valeurs intermédiaires sont interpolées, en particulier interpolées linéairement, si bien que les valeurs de base respectives de l'autre paramètre d'éclairage pertinent pour le spectre du jour prédéfini et les valeurs de base respectives de l'autre paramètre d'éclairage pertinent pour le spectre de l'autre jour respectif sont représentées par axe et la routine quotidienne respective de l'autre jour (d_j) résulte de la compression, de l'étirement ou du déplacement par section de la courbe de référence correspondante de la routine quotidienne respective du jour prédéfini et
- en plus de la commande ou du réglage de paramètres de fonctionnement du système d'éclairage pour le réglage de la routine quotidienne détectée du paramètre d'éclairage pertinent pour la luminosité φ' (t') et du paramètre d'éclairage pertinent pour le spectre λ' (t'), de plus commande ou réglage de paramètres de fonctionnement du système d'éclairage pour le réglage de la routine quotidienne détectée de l'autre paramètre d'éclairage pertinent pour le spectre (µ'(t), ..., v'(t)), respectivement pour chaque au moins autre jour (d_j).

3. Procédé selon l'une des revendications 1 et 2, **caractérisé en ce que** la multitude de temps de base et/ou la multitude de valeurs de base du paramètre d'éclairage pertinent pour la luminosité et/ou la multitude de valeurs de base du au moins un paramètre d'éclairage pertinent pour le spectre est fixée en fonction du lieu d'installation du système d'éclairage, respectivement pour au moins un autre jour.

4. Procédé selon l'une des revendications 1 à 3, **caractérisé en ce qu'**une multitude de routines quotidiennes d'utilisation spécifique du paramètre d'éclairage pertinent pour la luminosité (φ (t)) et d'au moins un paramètre d'éclairage pertinent pour le spectre (À (t), µ(t), ..., v(t))) pour le jour prédéfini (d_b) est mise à disposition pour la sélection.

5. Procédé selon l'une des revendications 1 à 4, **caractérisé en ce que** la multitude de temps de base et/ou la multitude de valeurs de base du paramètre d'éclairage pertinent pour la luminosité et/ou la multitude de valeurs de base d'au moins un paramètre d'éclairage pertinent pour le spectre est établie pour au moins un jour de la multitude de jours (d_j) répartis sur une année en fonction du lieu d'installation du système d'éclairage, en fonction de la saison sur le lieu d'installation du système d'éclairage et/ou en fonction de données météorologiques et/ou climatiques, en particulier de données météorologiques sur le lieu d'installation du système d'éclairage pour le au moins un autre jour (d_j).

6. Procédé selon l'une des revendications 1 à 5, **caractérisé en ce que** la multitude de temps de base et/ou la multitude de valeurs de base du paramètre d'éclairage pertinent pour la luminosité et/ou la multitude de valeurs de base d'au moins un paramètre d'éclairage pertinent pour le spectre est établie pour au moins un jour de la multitude de jours (d_j) répartis sur une année en fonction d'au moins une personne qui se trouve dans la zone d'action du système d'éclairage.

7. Procédé selon la revendication 6, **caractérisé en ce que** l'établissement est effectué sur la base de caractéristiques objectives de la au moins une personne comme l'âge, le sexe, le mouvement ou des caractéristiques subjectives ou la perception de la au moins une personne comme préférence de lumière, ambiance ou chronotype.

8. Procédé selon l'une des revendications 6 ou 7, **caractérisé en ce qu'**au moins une caractéristique objective ou subjective ou la perception de la au moins une personne est détectée au moyen d'un traitement de données de données recueillies par un dispositif de détection, en particulier des données d'image.

9. Procédé selon l'une des revendications 5 à 8, **caractérisé en ce que** la multitude de temps de base et/ou la multitude de valeurs de base du paramètre d'éclairage pertinent pour la luminosité et/ou la multitude de valeurs de base d'au moins un paramètre d'éclairage pertinent pour le spectre est fixée pour au moins un jour de la multitude de jours (d_j) répartis sur une année par une saisie d'utilisateur (Human-Machine-Interface), par un traitement de données de données détectées par un dispositif de détection et/ou de données recueillies par une connexion réseau.

10. Procédé selon l'une des revendications 1 à 9, **caractérisé en ce que** la fixation de la multitude de temps de base et/ou la multitude de valeurs de base du paramètre d'éclairage pertinent pour la luminosité et/ou la multitude de valeurs de base d'au moins un paramètre d'éclairage pertinent pour le spectre pour au moins un jour de la multitude de jours répartis sur une année se fait en fonction d'une multitude de données d'entrée.

11. Système d'éclairage comprenant un appareillage (2) pour alimenter au moins un moyen d'éclairage, un dispositif de commande (4) ainsi qu'une mémoire (8) pour stocker des courbes d'éclairage prédéfinies, cependant que le dispositif de commande est agencé et configuré pour déterminer, selon un procédé selon l'une des revendications 1 à 10, les routines quotidiennes respectives des paramètres d'éclairage et transmettre des signaux de commande correspondants à l'appareillage (2) pour régler le profil lumineux dans le système d'éclairage.
